# FASCICULE DE BREVET EUROPEEN

(11) **EP 1 355 989 B1**
(45) Date de publication et mention de la délivrance du brevet: **09.12.2009**
(21) Numéro de dépôt: 02710976.8
(22) Date de dépôt: 11.01.2002
(51) Int. Cl.: C08G 81/00

(54) **EMULSIONS ET COMPOSITIONS MOUSSANTES CONTENANT UN POLYMERE COMPRENANT DES UNITES HYDROSOLUBLES ET DES UNITES LCST, NOTAMMENT POUR DES APPLICATIONS COSMETIQUES**
SCHÄUMFÄHIGE ZUSAMMENZETZUNGEN UND EMULSIONEN ENTHALTEND EIN POLYMER MIT WASSERLÖSLICHEN UND LCST GRUPPEN, INSBESONDERE FÜR DIE KOSMETIK
FOAMING COMPOSITIONS AND EMULSIONS CONTAINING A POLYMER COMPRISING WATER-SOLUBLE UNITS AND LCST UNITS, PARTICULARLY FOR COSMETIC APPLICATIONS

(30) Priorité: 15.01.2001 FR 0100480
(43) Date de publication de la demande: 29.10.2003
(73) Titulaire: L'OREAL S.A., 75008 Paris Cedex (FR)
(72) Inventeur: L'ALLORET, Florence, F-75013 PARIS (FR)
(74) Mandataire: Poulin, Gérard
(86) Numéro de dépôt international: PCT/FR2002/000100
(87) Numéro de publication internationale: WO 2002/055606

(56) Documents cités:
- EP-A- 0 583 814
- EP-A- 0 629 649
- WO-A-95/24430
- WO-A-97/00275
- WO-A-98/29487
- WO-A-99/27924
- US-A- 5 939 485
- US-A- 6 159 457
- DURAND A ET AL: "Synthesis and thermoassociative properties in aqueous solution of graft copolymers containing poly(N-isopropylacrylamide) side chains" POLYMER, ELSEVIER SCIENCE PUBLISHERS B.V, GB, vol. 40, no. 17, août 1999 (1999-08), pages 4941-4951, XP004164960 ISSN: 0032-3861

## Description

### Domaine technique

La présente invention concerne une nouvelle utilisation de polymères comprenant des unités hydrosolubles et des unités à LCST, en particulier dans des émulsions et compositions moussantes notamment cosmétiques.

### Etat de la technique antérieure

Des polymères comportant des unités hydrosolubles et des unités à LCST ont été décrits dans les documents suivants : D. HOURDET et al., Polymer, 1994, vol. 35, n°12, pages 2624 à 2630 [1] ; F. L'ALLORET et al., Coll. Polym. Sci., 1995, vol. 273, n°12, pages 1163-1173, [2] ; F. L'ALLORET et al., Revue de l'Institut Français du Pétrole, 1997, vol. 52, n°2, pages 117-128 [3] ; EP-A-0 583 814 [4] et EP-A-0 629 649 [5].

Comme il est décrit dans ces documents, ces polymères comportent des unités hydrosolubles et des unités à LCST qui présentent dans l'eau une température inférieure critique de démixtion. Ainsi, ces unités à LCST sont des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" ("Lower Critical Solution Temperature"). Pour chaque concentration en polymère, une température de démixtion par chauffage est observée ; elle est supérieure à la LCST qui est le point minimum de la courbe. En dessous de cette température, le polymère est soluble dans l'eau, au-dessus de cette température, le polymère perd sa solubilité dans l'eau.

Aussi, ces polymères présentent des propriétés de gélification de l'eau provoquées par une augmentation de la température. Ces propriétés peuvent être mises à profit en vue d'applications dans le domaine pétrolier, comme il est décrit dans les documents [4] et [5].

WO-A-95/24430 [6] décrit également des copolymères comportant des unités à LCST et des unités sensibles au pH, qui présentent des propriétés de gélification en température. Ces copolymères, sont utilisés pour le relargage contrôlé de principes actifs dans le domaine pharmaceutique et dans le domaine cosmétique, sous forme de particules solides ou dans des formulations telles que des liquides, des gels ou des pommades. Les gels obtenus avec ces copolymères sont opaques et ont une LCST, contrairement aux polymères de l'invention qui comportent des unités LCST mais dont le comportement global n'est pas de type LCST et qui conduisent à des gels transparents.

US-A-5,939,485 [7] et WO 97/00275 [8] décrivent des systèmes polymères à gélification réversible comprenant un composant sensible capable d'agrégation en réponse à un changement dans un stimulus extérieur et un composant structural. Le stimulus extérieur peut être la température. Le composant sensible peut être un copolymère bloc tel qu'un Poloxamer, par exemple un Pluronic®, qui s'agrège au niveau microscopique au-delà d'une température critique ne correspondant pas à une LCST. On peut aussi utiliser comme composant sensible un tensio-actif non ionique. Ces polymères ont des propriétés de gélification par chauffage et ils peuvent être utilisés dans le domaine pharmaceutique pour la délivrance de médicaments et dans de nombreux autres domaines y compris le domaine des cosmétiques. L'exemple 34 de WO 97/00275 [8] illustre des formulations cosmétiques comprenant le système poloxamer-dérivé acrylique avec addition de tensio-actifs non ioniques, anioniques et cationiques.

Dans ces formulations, le composant sensible du système polymère a un comportement différent de celui d'unités à LCST lors du chauffage. Ainsi, lorsqu'on le chauffe à environ 30-40°C, il présente une température de micellisation, c'est-à-dire une agrégation à l'échelle microscopique, puis lorsqu'on le chauffe davantage, une température de LCST supérieure. Cette LCST correspond à une agrégation à l'échelle macroscopique entre les macromolécules. Il est expliqué dans WO-A-97/00275 [8] aux pages 16 et 17, que la gélification et la LCST sont observées à des températures qui diffèrent d'environ 70°C. Ceci montre que ces polymères sont différents de ceux de notre demande.

On connaît encore par le document WO-A-98/48768 [9] des compositions cosmétiques utilisant un système polymère à thermogélification réversible, comprenant l'acide polyacrylique et un poloxamer. Ce polymère est donc différent des polymères de l'invention.

WO-A-00/35961 [10] décrit la préparation de polymères ayant des propriétés d'épaississement en température par polymérisation en émulsion et l'utilisation de ces polymères dans des compositions pharmaceutiques et cosmétiques. Ces polymères peuvent être des copolymères ayant des unités hydrosolubles et des unités à LCST à base d'oxyde d'alkylène. Il est prévu d'ajouter des tensio-actifs non ioniques aux polymères pour renforcer leurs propriétés thermo-épaississantes.

Ainsi, il ressort de ces documents que des polymères comportant des unités hydrosolubles et des unités à LCST ont des propriétés thermogélifiantes ou thermo-épaississantes.

### Exposé de l'invention

Selon l'invention, on a découvert que des polymères comprenant des unités hydrosolubles et des unités à LCST, les unités à LCST présentant dans l'eau une température de démixtion de 5 à 40°C à une concentration massique de 1% dans l'eau, présentaient de plus la propriété avantageuse d'abaisser la tension superficielle ou la tension interfaciale de l'eau, et de pouvoir être utilisés de ce fait pour la fabrication de compositions moussantes et d'émulsions.

Aussi, l'invention a pour objet l'utilisation d'un polymère comprenant des unités hydrosolubles et des unités à LCST, les unités à LCST présentant dans l'eau une température de démixtion de 5 à 40°C à une concentration massique de 1% dans l'eau, pour abaisser la tension superficielle ou la tension interfaciale de l'eau.

Selon une caractéristique avantageuse de l'invention, l'abaissement de la tension superficielle ou de la tension interfaciale de l'eau est d'au moins 15 mN/m pour une concentration massique en polymère dans l'eau de 0,1% dans la gamme de température allant de 5 à 80°C.

Par ailleurs, cet effet d'abaissement de la tension superficielle ou de la tension interfaciale de l'eau est renforcé lorsque la température devient supérieure à la température de démixtion des unités à LCST. Dans ce cas, l'abaissement de la tension superficielle ou de la tension interfaciale de l'eau est d'au moins 20 mN/m pour une concentration massique en polymère dans l'eau de 0,1% lorsque la température est supérieure à la température de démixtion des unités à LCST à cette concentration.

Ces propriétés interfaciales des polymères comprenant des unités hydrosolubles et des unités à LCST peuvent être mises à profit pour la fabrication de mousses ou d'émulsions huile-dans-eau ou multiple eau-dans huile-dans eau (E/H/E), sans tensio-actif ou contenant une très faible teneur en tensio-actif.

Les mousses sont des dispersions de bulles de gaz, en particulier d'air, dans une phase continue aqueuse. Dans les compositions cosmétiques nettoyantes, la stabilisation de ces bulles est assurée par des tensio-actifs dont la concentration massique varie de 5% à 20%. Ces espèces amphiphiles de faible masse molaire (M<2000 g/mole) présentent le désavantage d'avoir un caractère relativement agressif vis-à-vis de la peau.

Les émulsions huile-dans-eau (H/E) sont des dispersions de globules huileux dans une phase continue aqueuse. La stabilisation des émulsions cosmétiques H/E ou des émulsions multiples E/H/E peut être assurée par des espèces amphiphiles de différentes natures :
- les tensio-actifs, se caractérisant par leur faible masse molaire (<2000 g/mole),
- les gélifiants amphiphiles de type microgels tels que le Pemulen TR1 (Goodrich), et
- les dérivés diméthicone copolyols utilisés en présence d'huile siliconée.

Chacun de ces composés présente des limites d'utilisation :
- les tensio-actifs possèdent un caractère relativement agressif vis-à-vis de la peau ;
- les gélifiants amphiphiles de type microgels sont en nombre restreint et conduisent tous à des textures gélifiées de même type,
- les dérivés diméthicone copolyols sont spécifiques aux huiles siliconées.

D'autre part, la stabilité des émulsions est généralement diminuée lorsque la température augmente, ce qui peut conduire à des phénomènes de déphasage lors du stockage des émulsions en raison des variations de température.

Aussi, pour des applications cosmétiques, il est intéressant de disposer d'agents :
- permettant de stabiliser des mousses et émulsions cosmétiques, notamment sur une large gamme de température (5°C-80°C),
- peu agressifs pour la peau, et
- pouvant conduire à une large gamme de textures, fluides ou épaissies/gélifiées.

Selon l'invention, on a trouvé que les polymères comprenant des unités hydrosolubles et des unités à LCST présentant des températures de démixtion appropriées, permettaient d'obtenir des mousses et des émulsions sans tensio-actif ou en contenant une très faible teneur, donc peu agressives pour la peau, stables dans la gamme de température allant de 5 à 80°C et pouvant conduire à une large gamme de textures.

Aussi, l'invention a également pour objet une composition moussante comprenant une phase aqueuse contenant un polymère comprenant des unités hydrosolubles et des unités à LCST, les unités à LCST présentant dans l'eau une température de démixtion de 5 à 40°C à une concentration massique de 1% dans l'eau.

Elle concerne encore une émulsion huile-dans-eau comprenant une phase aqueuse et une phase huileuse dispersée dans la phase aqueuse, dans laquelle la phase aqueuse comprend un polymère comprenant des unités hydrosolubles et des unités à LCST, les unités à LCST présentant dans l'eau une température de démixtion de 5 à 40°C à une concentration massique de 1% dans l'eau.

L'invention concerne aussi une émulsion eau-dans-huile-dans eau comprenant une émulsion eau-dans huile dispersée dans une phase aqueuse externe, dans laquelle la phase aqueuse externe comprend un polymère comprenant des unités hydrosolubles et des unités à LCST, les unités à LCST présentant dans l'eau une température de démixtion de 5 à 40°C à une concentration massique de 1% dans l'eau.

Les polymères utilisés dans l'invention peuvent être des polymères séquencés (ou blocs), ou des polymères greffés, qui comprennent, d'une part, des unités hydrosolubles et, d'autre part, des unités à LCST telles que définies ci-dessus.

Les polymères, employés dans le cadre de l'invention peuvent donc être des polymères séquences, comprenant par exemple des séquences hydrosolubles alternées avec des séquences à LCST.

Ces polymères peuvent également se présenter sous la forme de polymères greffés dont le squelette est formé d'unités hydrosolubles, porteur de greffons à LCST. Ces polymères peuvent également se présenter sous la forme de polymères greffés dont le squelette est formé d'unités à LCST, porteur de greffons hydrosolubles.

Ces structures peuvent être partiellement réticulées.

On précise que dans le présent texte, les termes "unité hydrosoluble" et "unité à LCST" n'incluent pas les groupes liant entre elles, d'une part, les unités hydrosolubles et, d'autre part, les unités à LCST, les groupes de liaison étant issus de la réaction des sites réactifs portés, d'une part, par des précurseurs des unités hydrosolubles et, d'autre part, par des précurseurs des unités à LCST.

Les unités hydrosolubles de ces polymères sont des unités solubles dans l'eau, à une température de 5°C à 80°C, à raison d'au moins 10 g/l, de préférence d'au moins 20 g/l.

Toutefois, on entend également par unités hydrosolubles, des unités ne possédant pas obligatoirement la solubilité ci-dessus mentionnée, mais qui en solution aqueuse à 1% en poids, de 5°C à 80°C, permettent l'obtention d'une solution macroscopiquement homogène et transparente, c'est-à-dire ayant une valeur de transmittance maximum de la lumière, quelle que soit la longueur d'onde comprise entre 400 et 800 nm, à travers un échantillon de 1 cm d'épaisseur, d'au moins 85%, de préférence d'au moins 90%.

Ces unités hydrosolubles ne présentent pas de température de démixtion par chauffage de type LCST.

Ces unités hydrosolubles peuvent être obtenues par polymérisation radicalaire de monomères vinyliques, ou par polycondensation, ou encore peuvent être constituées par des polymères naturels ou naturels modifiés existants.

A titre d'exemple, on peut citer les monomères suivants, qui sont susceptibles d'être employés pour former lesdites unités hydrosolubles, seuls ou en mélange :
- l'acide (méth)acrylique ;
- les monomères vinyliques de formule (I) suivante : 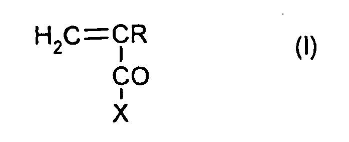
dans laquelle :
- R est choisi parmi H, -CH₃, -C₂H₅ ou -C₃H₇, et
- X est choisi parmi :
   - les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor) ; un groupement sulfonique (-SO₃⁻), sulfate (-SO₄⁻), phosphate (-P0₄H₂) ; hydroxy (-OH) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
   - les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂); amine primaire (-NH₂) ; amine secondaire (-NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
- l'anhydride maléique ;
- l'acide itaconique ;
- l'alcool vinylique de formule CH₂=CHOH ;
- l'acétate de vinyle de formule CH₂=CH-OCOCH₃ ;
- les N-vinyllactames tels que la N-vinylpyrrolidone, la N-vinylcaprolactame et la N-butyrolactame ;
- les vinyléthers de formule CH₂=CHOR₆ dans laquelle R₆ est un radical hydrocarboné, linéaire ou ramifié, saturé ou insaturé, ayant de 1 à 6 carbones ;
- les dérivés hydrosolubles du styrène, notamment le styrène sulfonate ;
- le chlorure de diméthyldiallyl ammonium ; et
- la vinylacétamide.

Parmi les polycondensats et les polymères naturels ou naturels modifiés susceptibles de constituer tout ou partie des unités hydrosolubles, on peut citer :
- les polyuréthanes hydrosolubles,
- la gomme de xanthane, notamment celle commercialisée sous les dénominations Keltrol T et Keltrol SF par Kelco ; ou Rhodigel SM et Rhodigel 200 de Rhodia ;
- les alginates (Kelcosol de Monsanto) et leurs dérivés tels que l'alginate de propylène glycol (Kelcoloid LVF de Kelco) ;
- les dérivés de cellulose et notamment la carboxyméthylcellulose (Aquasorb A500, Hercules), l'hydroxypropylcellulose, l'hydroxyéthylcellulose et l'hydroxyéthylcellulose quaternisée ;
- les galactomannanes et leurs dérivés, tels que la gomme Konjac, la gomme de guar, l'hydroxypropylguar, l'hydroxypropylguar modifié par des groupements méthylcarboxylate de sodium (Jaguar XC97-1, Rhodia), le chlorure de guar hydroxypropyl tri-méthyl ammonium.

On peut également citer la polyéthylène imine.

De préférence, les unités hydrosolubles ont une masse molaire allant de 1000 g/mole à 5 000 000 g/mole lorsqu'elles constituent le squelette hydrosoluble d'un polymère greffé.

Ces unités hydrosolubles ont de préférence une masse molaire allant de 500 g/mole à 100 000 g/mole lorsqu'elles constituent un bloc d'un polymère multiblocs ou bien lorsqu'elles constituent les greffons d'un polymère greffé ou en peigne.

On peut définir les unités à LCST des polymères utilisés dans l'invention, comme étant des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" (Lower Critical Solution Temperature). Pour chaque concentration en polymère, une température de démixtion par chauffage est observée ; elle est supérieure à la LCST qui est le point minimum de la courbe. En dessous de cette température, le polymère est soluble dans l'eau ; au dessus de cette température, le polymère perd sa solubilité dans l'eau.

Par soluble dans l'eau à la température T, on entend que les unités présentent une solubilité à T, d'au moins 1 g/l, de préférence d'au moins 2 g/l.

La mesure de la LCST peut se faire visuellement : on détermine la température à laquelle apparaît le point de trouble de la solution aqueuse ; ce point de trouble se traduit par l'opacification de la solution, ou perte de transparence.

D'une manière générale, une composition transparente aura une valeur de transmittance maximum de la lumière, quelle que soit la longueur d'onde comprise entre 400 et 800 nm, à travers un échantillon de 1 cm d'épaisseur, d'au moins 85%, de préférence d'au moins 90%.

La transmittance peut être mesurée en plaçant un échantillon de 1 cm d'épaisseur dans le rayon lumineux d'un spectrophotomètre travaillant dans les longueurs d'onde du spectre lumineux.

Les unités à LCST des polymères utilisés dans l'invention peuvent être constituées d'un ou plusieurs des polymères suivants :
- les polyéthers tels que le poly oxyde d'éthylène (POE), le poly oxyde de propylène (POP), les copolymères statistiques d'oxyde d'éthylène (OE) et d'oxyde de propylène (OP),
- le polyvinylméthyléther,
- le polyN-isopropylacrylamide et le polyN-éthylacrylamide, et
- le polyvinylcaprolactame.

De préférence, les unités à LCST sont constituées de polyoxyde de propylène (POP)ₙ avec n étant un nombre entier de 10 à 50, ou de copolymères statistiques d'oxyde d'éthylène (OE) et d'oxyde de propylène (OP), représentés par la formule :

(OE)ₘ (OP)ₙ

dans laquelle m est un nombre entier allant de 1 à 40, de préférence de 2 à 20, et n est un nombre entier allant de 10 à 60, de préférence de 20 à 50.

De préférence, la masse molaire de ces unités à LCST est de 500 à 5300 g/mole, notamment de 1500 à 4000 g/mole.

On a constaté que la répartition statistique des motifs OE et OP se traduit par l'existence d'une température inférieure critique de démixtion, au-delà de laquelle une séparation de phases macroscopique est observée. Ce comportement est différent de celui des copolymères (OE) (OP) à blocs, qui micellisent au delà d'une température critique dite de micellisation (agrégation à l'échelle microscopique).

Les unités à LCST peuvent donc notamment être issues de copolymères statistiques d'oxyde d'éthylène et d'oxyde de propylène, aminés, notamment monoaminés, diaminés ou triaminés. Parmi les unités à LCST commercialement disponibles, on peut citer les copolymères vendus sous la dénomination Jeffamine par HUNTSMAN, et notamment la Jeffamine XTJ-507 (M-2005), la Jeffamine D-2000 et la Jeffamine XTJ-509 (ou T-3000).

Les unités à LCST peuvent également être issues de copolymères OE/OP statistiques à extrémités OH, tels que ceux vendus sous la dénomination Polyglycols P41 et B11 par Clariant.

On peut aussi utiliser dans l'invention comme unités à LCST, les dérivés polymériques et copolymériques N-substitués de l'acrylamide, ayant une LCST, ainsi que le polyvinyl caprolactame et les copolymères de vinylcaprolactame.

A titre d'exemples de dérivés polymériques N-substitués de l'acrylamide ayant une LCST, on peut citer le poly N-isopropylacrylamide, le poly N-éthylacrylamide et les copolymères de N-isopropylacrylamide (ou de N-éthylacrylamide) et d'un monomère vinylique ayant la formule (I) donnée ci-dessus, ou d'un monomère choisi parmi l'anhydride maléique, l'acide itaconique, la vinylpyrrolidone, le styrène et ses dérivés, le chlorure de diméthyldiallyl ammonium, la vinylacétamide, les vinyléthers et les dérivés de l'acétate de vinyle.

La masse molaire de ces polymères est de préférence de 1 000 g/mole à 500 000 g/mole, de préférence de 2 000 à 50 000 g/mole.

La synthèse de ces polymères peut être réalisée par polymérisation radicalaire à l'aide d'un couple d'amorceurs tel que le chlorhydrate d'aminoéthanethiol, en présence de persulfate de potassium, afin d'obtenir des unités LCST ayant une extrémité réactive aminée.

A titre d'exemples de copolymères de vinylcaprolactame, on peut citer les copolymères de vinyl caprolactame et d'un monomère vinylique ayant la formule (I) donnée ci-dessus, ou d'un monomère choisi parmi l'anhydride maléique, l'acide itaconique, la vinylpyrrolidone, le styrène, et ses dérivés, le chlorure de diméthyldiallyl ammonium, la vinylacétamide, l'alcool vinylique, l'acétate de vinyle, les vinyléthers et les dérivés de l'acétate de vinyle.

La masse molaire de ces polymères ou copolymères de vinylcaprolactame est généralement de 1 000 g/mole à 500 000 g/mole, de préférence de 2 000 à 50 000 g/mol.

La synthèse de ces composés peut être réalisée par polymérisation radicalaire à l'aide d'un couple d'amorceurs tel que le chlorhydrate d'aminoéthanethiol, en présence de persulfate de potassium, afin d'obtenir des unités LCST ayant une extrémité réactive aminée.

La proportion massique des unités à LCST dans le polymère final est de préférence de 5% à 70%, notamment de 20% à 65%, et particulièrement de 30% à 60% en poids, par rapport au polymère final.

Comme défini précédemment, la température de démixtion desdites unités à LCST est de 5°C à 40°C, de préférence elle est de 10°C à 35°C, pour une concentration massique dans l'eau, de 1% en poids desdites unités à LCST.

Les polymères employés dans le cadre de l'invention peuvent être aisément préparés par l'homme du métier sur la base de ses connaissances générales, en utilisant des procédés de greffage, de copolymérisation ou de réaction de couplage.

Lorsque le polymère final se présente sous la forme d'un polymère greffé, notamment présentant un squelette hydrosoluble avec des chaînes latérales à LCST, il est possible de le préparer par greffage des unités à LCST ayant au moins une extrémité réactive, notamment aminée, sur un polymère hydrosoluble formant le squelette, portant au minimum 10% (en mole) de groupes réactifs tels que des fonctions acides carboxyliques. Cette réaction peut s'effectuer en présence d'un carbodiimide tel que le dicyclohexylcarbodiimide ou le chlorhydrate de 1-(3-diméthylaminopropyl)-3-éthyl carbodiimide, dans un solvant tel que la N-méthylpyrrolidone ou l'eau.

Une autre possibilité pour préparer des polymères greffés consiste à copolymériser par exemple un macromonomère à LCST (chaîne à LCST précédemment décrite avec une extrémité vinylique) et un monomère vinylique hydrosoluble tel que l'acide acrylique ou les monomères vinyliques ayant la formule (I).

Lorsque le polymère final se présente sous la forme d'un polymère à blocs, il est possible de le préparer par couplage entre des unités hydrosolubles et des unités à LCST, ces unités ayant à chaque extrémité des sites réactifs complémentaires.

Dans le cas des procédés de greffage et des procédés de couplage, les sites réactifs des unités à LCST peuvent être des fonctions amines notamment monoamines, diamines ou triamines, et des fonctions OH. Dans ce cas, les sites réactifs des unités hydrosolubles peuvent être des fonctions acides carboxyliques.

Comme on l'a vu précédemment, les compositions moussantes de l'invention comportent une phase aqueuse contenant un polymère comprenant des unités hydrosolubles et des unités à LCST, tel que défini ci-dessus. Généralement, la concentration massique en polymère de la phase aqueuse est égale ou inférieure à 5%, de préférence de 0,01 à 5% et mieux de 0,02 à 4% du poids de la phase aqueuse.

La phase aqueuse peut comprendre de plus un tensio-actif moussant en faible quantité, par exemple à une concentration massique égale ou inférieure à 5%, de préférence égale ou inférieure à 4% en poids par rapport au poids de phase aqueuse.

Les tensio-actifs moussants utilisés peuvent être des tensioactifs non ioniques, anioniques, amphotères ou zwitterioniques. De préférence, le ou les tensio-actifs moussants sont choisis parmi les tensio-actifs non ioniques.

Comme tensioactifs non ioniques, on peut citer par exemple les alkylpolyglycosides (APG), les esters de polyols et d'acides gras, les esters de polyéthylène glycols et d'acide gras, les dérivés d'alcools gras et de polyols (éthers), et les dérivés oxyalkylénés (oxyéthylénés et/ou oxypropylénés) de ces composés. On peut citer aussi les esters de maltose, les alcools gras polyglycérolés, les dérivés de glucamine comme l'éthyl-2 hexyl oxycarbonyl N-méthyl glucamine, et leurs mélanges.

Comme alkylpolyglucosides, on peut citer par exemple le décylglucoside (Alkyl-C9/C11-polyglucoside (1.4)) comme le produit commercialisé sous la dénomination MYDOL 10 par la société Kao Chemicals, le produit commercialisé sous la dénomination PLANTAREN 2000 UP et PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel, et leurs mélanges.

Les dérivés de maltose sont par exemple ceux décrits dans le document EP-A-566438 [11], tels que l'O-octanoyl-6'-D-maltose, ou encore le O-dodécanoyl-6'-D-maltose décrit dans le document FR-2,739,556 [12].

Parmi les alcools gras polyglycérolés, on peut citer le dodécanediol polyglycérolé (3,5 moles de glycérol), produit commercialisé sous la dénomination CHIMEXANE NF par la société Chimex.

Comme tensioactifs anioniques, on peut utiliser par exemple les carboxylates, les dérivés des aminoacides, les alkyl sulfates, les alkyl éther sulfates, les sulfonates, les iséthionates, les taurates, les sulfosuccinates, les alkylsulfoacétates, les phosphates et alkylphosphates, les polypeptides, les dérivés anioniques d'alkyl polyglucoside, les savons d'acides gras, et leurs mélanges.

Comme carboxylates, on peut citer par exemple les sels alcalins de N-acylaminoacides ; les amidoéthercarboxylates (AEC) comme le lauryl amidoéther carboxylate de sodium (3 OE) commercialisé sous la dénomination AKYPO FOAM 30 par la société Kao Chemicals ; les sels d'acides carboxyliques polyoxyéthylénés, comme le lauryl éther carboxylate de sodium (C12-14-16 65/25/10) oxyéthyléné (6 OE) commercialisé sous la dénomination AKYPO SOFT 45 NV par la société Kao Chemicals ; les acides gras d'huile d'olive polyoxyéthylénés et de carboxyméthyl, comme le produit commercialisé sous la dénomination OLIVEM 400 par la société Biologia E Tecnologia ; le tri-décyl éther carboxylate de sodium oxyéthyléné (6 OE) commercialisé sous la dénomination NIKKOL ECTD-6NEX par la société Nikkol ; le 2-(2-Hydroxyalkyloxy) acétate de sodium commercialisé sous la dénomination BEAULIGHT SHAA par la société SANYO.

Les dérivés des aminoacides peuvent être choisis par exemple parmi les sarcosinates et notamment les acylsarcosinates comme le lauroylsarcosinate de sodium commercialisé sous la dénomination SARKOSYL NL 97 par la société Ciba ou commercialisé sous la dénomination ORAMIX L 30 par la société Seppic, le myristoyl sarcosinate de sodium, commercialisé sous la dénomination NIKKOL SARCOSINATE MN par la société Nikkol, le palmitoyl sarcosinate de sodium, commercialisé sous la dénomination NIKKOL SARCOSINATE PN par la société Nikkol ; les alaninates comme le N-lauroyl-N-méthylamidopropionate de sodium commercialisé sous la dénomination SODIUM NIKKOL ALANINATE LN 30 par la société Nikkol ou commercialisé sous la dénomination ALANONE ALE par la société Kawaken, et le N-lauroyl N-méthylalanine triéthanolamine, commercialisé sous la dénomination ALANONE ALTA par la société Kawaken ; les N-acylglutamates comme le mono-cocoylglutamate de triéthanolamine commercialisé sous la dénomination ACYLGLUTAMATE CT-12 par la société Ajinomoto, le lauroyl-glutamate de triéthanolamine commercialisé sous la dénomination ACYLGLUTAMATE LT-12 par la Société Ajinomoto et le N-lauroyl-L-glutamate monosodique commercialisé sous la dénomination AMISOFT LS-11 par la société Ajinomoto ; les aspartates comme le mélange de N-lauroylaspartate de triéthanolamine et de N-myristoylaspartate de triéthanolamine, commercialisé sous la dénomination ASPARACK LM-TS2 par la société Mitsubishi ; les citrates, et leurs mélanges.

Comme dérivés de la glycine, on peut citer le N-cocoylglycinate de sodium et le N-cocoylglycinate de potassium comme les produits commercialisés sous les dénominations AMILITE GCS-12 et AMILITE GCK-12 par la société Ajinomoto.

Comme alkyl éther sulfates, on peut citer par exemple le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel, l'alkyl (C12-C14) éther (9 OE) sulfate d'ammonium commercialisé sous la dénomination RHODAPEX AB/20 par la société Rhodia Chimie.

Comme sulfonates, ôn peut citer par exemple les alpha-oléfines sulfonates comme l'alpha-oléfine sulfonate de sodium (C14-16) commercialisé sous la dénomination BIO-TERGE AS-40 par la société Stepan, commercialisé sous les dénominations WITCONATE AOS PROTEGE et SULFRAMINE AOS PH 12 par la société Witco ou commercialisé sous la dénomination BIO-TERGE AS-40 CG par la société Stepan, l'oléfine sulfonate de sodium secondaire commercialisé sous la dénomination HOSTAPUR SAS 30 par la société Clariant ; les alkyl aryl sulfonates linéaires comme le xylène sulfonate de sodium commercialisé sous les dénominations MANROSOL SXS30, MANROSOL SXS40, MANROSOL SXS93 par la société Manro.

Comme iséthionates, on peut citer les acyliséthionates comme le cocoyl-iséthionate de sodium, tel que le produit commercialisé sous la dénomination JORDAPON CI P par la société Jordan.

Comme taurates, on peut citer le sel de sodium de méthyltaurate d'huile de palmiste commercialisé sous la dénomination HOSTAPON CT PATE par la société Clariant ; les N-acyl N-méthyltaurates comme le N-cocoyl N-méthyltaurate de sodium commercialisé sous la dénomination HOSTAPON LT-SF par la société Clariant ou commercialisé sous la dénomination NIKKOL CMT-30-T par la société Nikkol, le palmitoyl méthyltaurate de sodium commercialisé sous la dénomination NIKKOL PMT par la société Nikkol.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL, REWOPOL SB-FA 30 K 4 par la société Witco, le sel di-sodique d'un hémi-sulfosuccinate des alcools C12-C14, commercialisé sous la dénomination SETACIN F SPECIAL PASTE par la société Zschimmer Schwarz, l'oléamidosulfosuccinate di-sodique oxyéthyléné (2 OE) commercialisé sous la dénomination STANDAPOL SH 135 par la société Henkel, le mono-sulfosuccinate d'amide laurique oxyéthyléné (5 OE) commercialisé sous la dénomination LEBON A-5000 par la société Sanyo, le sel di-sodique de mono-sulfosuccinate de lauryl citrate oxyéthyléné (10 OE) commercialisé sous la dénomination REWOPOL SB CS 50 par la société Witco, le mono-sulfosuccinate de mono-éthanolamide ricinoléique commercialisé sous la dénomination REWODERM S 1333 par la société Witco.

Comme phosphates et alkylphosphates, on peut citer par exemple les monoalkylphosphates et les dialkylphosphates, tels que le monophosphate de lauryle commercialisé sous la dénomination MAP 20 par la société Kao Chemicals, le sel de potassium de l'acide dodécylphosphorique, mélange de mono- et di-ester (diester majoritaire) commercialisé sous la dénomination CRAFOL AP-31 par la société Cognis, le mélange de monoester et de di-ester d'acide octylphosphorique, commercialisé sous la dénomination CRAFOL AP-20 par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER par la société Condea, le sel de potassium ou de triéthanolamine de mono-alkyl (C12-C13) phosphate commercialisé sous les références ARLATONE MAP 230K-40 et ARLATONE MAP 230T-60 par la société Uniqema, le lauryl phosphate de potassium commercialisé sous la dénomination DERMALCARE MAP XC-99/09 par la société Rhodia Chimie.

Les polypeptides sont obtenus par exemple par condensation d'une chaîne grasse sur les aminoacides de céréale et notamment du blé et de l'avoine. Comme polypeptides, on peut citer par exemple le sel de potassium de la lauroyl protéine de blé hydrolysée, commercialisé sous la dénomination AMINOFOAM W OR par la société Croda, le sel de triéthanolamine de cocoyl protéine de soja hydrolysée, commercialisé sous la dénomination MAY-TEIN SY par la société Maybrook, le sel de sodium des lauroyl amino-acides d'avoine, commercialisé sous la dénomination PROTEOL OAT par la société Seppic, l'hydrolysat de collagène greffé sur l'acide gras de coprah, commercialisé sous la dénomination GELIDERM 3000 par la société Deutsche Gelatine, les protéines de soja acylées par des acides de coprah hydrogénés, commercialisées sous la dénomination PROTEOL VS 22 par la société Seppic.

Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET par la société Cesalpinia, le sel di-sodique d'ester sulfosuccinique de cocoylpolyglucoside (1,4), commercialisé sous la dénomination ESSAI 512 MP par la société Seppic, le sel de sodium d'ester citrique de cocoyl polyglucoside (1,4) commercialisé sous la dénomination EUCAROL AGE-EC^{®} par la société Cesalpinia. Un autre dérivé d'holoside anionique peut être le dodécyl-D-galactoside uronate de sodium commercialisé sous la dénomination DODECYL-D-GALACTOSIDE URONATE DE SODIUM par la société SOLIANCE.

Les savons d'acide gras qui peuvent être utilisés comme tensioactifs anioniques sont des acides gras d'origine naturelle ou synthétique, salifiés par une base minérale ou organique. La chaîne grasse peut comporter de 6 à 22 atomes de carbone, de préférence de 8 à 18 atomes de carbone. La base minérale ou organique peut être choisie parmi les métaux alcalins ou alcalino-terreux, les aminoacides et les aminoalcools. Comme sels, on peut utiliser par exemple les sels de sodium, de potassium, de magnésium, de triéthanolamine, de N-méthylglucamine, de lysine et d'arginine. Comme savons, on peut citer par exemple les sels de potassium ou de sodium des acides laurique, myristique, palmitique, stéarique (laurate, myristate, palmitate et stéarate de potassium ou de sodium), et leurs mélanges.

Comme tensioactifs amphotères et zwitterioniques, on peut utiliser par exemple, les bétaïnes, les N-alkylamidobétaines et leurs dérivés, les dérivés de la glycine, les sultaïnes, les alkyl polyaminocarboxylates, les alkylamphoacétates et leurs mélanges.

Comme bétaïnes, on peut citer par exemple la cocobétaïne comme le produit commercialisé sous la dénomination DEHYTON AB-30 par la société Henkel, la laurylbétaïne comme le produit commercialisé sous la dénomination GENAGEN KB par la société Clariant, la laurylbétaïne oxyéthylénée (10 OE), comme le produit commercialisé sous la dénomination LAURYLETHER(10 OE)BETAINE par la société Shin Nihon Rica, la stéarylbétaïne oxyéthylénée (10 OE) comme le produit commercialisé sous la dénomination STEARYLETHER (10 OE)BETAINE par la société Shin Nihon Rica.

Parmi les N-alkylamidobétaines et leurs dérivés, on peut citer par exemple la cocamidopropyl bétaine commercialisée sous la dénomination LEBON 2000 HG par la société Sanyo, ou commercialisée sous la dénomination EMPIGEN BB par la société Albright & Wilson, la lauramidopropyl bétaïne commercialisée sous la dénomination REWOTERIC AMB12P par la société Witco.

Comme sultaïnes, on peut citer le cocoylamidopropylhydroxy-sulfobétaine commercialisé sous la dénomination CROSULTAINE C-50 par la société Croda.

Comme alkyl polyaminocarboxylates (APAC), on peut citer le cocoylpolyamino-carboxylate de sodium, commercialisé sous la dénomination AMPHOLAK 7 CX/C,et AMPHOLAK 7 CX par la société Akzo Nobel, le stéarylpolyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP par la société Rhodia Chimie, et le N-cocoyl-N-hydroxyéthyl-N-carboxyméthyl-éthylènediamine N-sodique (nom CTFA : sodium cocamphoacétate).

Parmi ces tensioactifs, ceux pouvant se présenter sous forme de poudre sont par exemple le cocoylisethionate de sodium, le mono-sulfosuccinate de mono-éthanolamide ricinoléique commercialise sous la dénomination REWODERM S 1333 par la société Witco, le N-lauroyl-L-glutamate monosodique (Amisoft LS-11), et le palmitoyl méthyltaurate de sodium commercialisé sous la dénomination NIKKOL PMT par la société Nikkol.

Dans le cas des émulsions huile-dans-eau de l'invention, la phase aqueuse comprend un polymère comprenant des unités hydrosolubles et des unités à LCST, tel que défini ci-dessus et la concentration massique en polymère de la phase aqueuse est inférieure ou égale à 5%, de préférence de 0,01 à 5% et mieux 0,02 à 4% en poids de phase aqueuse.

La phase aqueuse peut contenir de plus un tensio-actif émulsionnant additionnel en quantité très faible, ne dépassant pas 1%.

Comme tensio-actifs émulsionnants, on peut citer notamment des émulsionnants non-ioniques et par exemple les produits d'addition de 1 à 200 moles d'oxyde d'éthylène ou d'oxyde de propylène sur des esters partiels de polyols ayant 2 à 16 atomes de carbone et d'acides gras ayant 12 à 22 atomes de carbone comme les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween^{®} 20 ou Tween^{®} 60, les esters de sucres comme le stéarate de sucrose, et leurs mélanges.

Dans les compositions moussantes et les émulsions de l'invention, la phase aqueuse peut être constituée par un milieu physiologiquement acceptable permettant une application topique et notamment cosmétique.

On entend dans la présente demande par "milieu physiologiquement acceptable" un milieu compatible avec toutes les matières kératiniques telles que la peau y compris le cuir chevelu, les ongles, les muqueuses, les yeux et les cheveux ou toute autre zone cutanée du corps.

Le milieu physiologiquement acceptable des émulsions et compositions moussantes de l'invention comprend de l'eau. La quantité d'eau peut aller de 20 à 99,98% en poids et de préférence de 40 à 95 % en poids par rapport au poids total de la composition.

L'eau utilisée peut être outre de l'eau, une eau florale telle que l'eau de bleuet, une eau minérale telle que l'eau de Vittel, l'eau de LUCAS ou l'eau de la Roche Posay et/ou une eau thermale.

Le milieu physiologiquement acceptable peut contenir, outre l'eau, un ou plusieurs solvants choisis parmi les alcools inférieurs comportant de 1 à 8 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose et le sucrose ; et leurs mélanges. La quantité de solvant (s) peut aller de 0,5 à 30% en poids et de préférence de 5 à 20% en poids par rapport au poids total de la composition.

La phase huileuse présente dans les émulsions et éventuellement présente dans les compositions moussantes peut être constituée de tous les corps gras utilisés dans le domaine cosmétique.

La phase huileuse comporte de préférence au moins une huile.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate; le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- des huiles essentielles naturelles ou synthétique telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, et les polyméthylphénylsiloxanes ;
- leurs mélanges.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations "KSG" par la société Shin-Etsu, sous les dénominations "Trefil", "BY29" ou "EPSX" par la société Dow Corning ou sous les dénominations "Gransil" par la société Grant Industries.

Ces corps gras peuvent être choisis de manière variée par l'homme du métier afin de préparer une composition ayant les propriétés, par exemple de consistance ou de texture, souhaitées.

Quand elle est présente, la quantité de phase huileuse peut aller par exemple de 0,01 à 50 % en poids et de préférence de 0,1 à 30 % en poids par rapport au poids total de la composition.

Les émulsions et compositions moussantes de l'invention peuvent encore contenir des adjuvants habituels dans les domaines cosmétique et dermatologique, tels que les charges minérales ou organiques, les actifs hydrophiles ou lipophiles, les conservateurs, les gélifiants, les plastifiants, les antioxydants, les parfums, les absorbeurs d'odeur, les filtres UV, les séquestrants (EDTA), les ajusteurs de pH acides ou basiques ou des tampons, et des matières colorantes (pigments ou colorants ou nacres). Dans le cas des émulsions, ces adjuvants, selon leur nature, peuvent être introduits dans la phase huileuse, dans la phase aqueuse et/ou dans les vésicules lipidiques. Les quantités de ces différents additifs sont celles classiquement utilisées dans les domaines considérés, et par exemple de 0,01 à 20% du poids total de la composition. Bien entendu, l'homme de l'art veillera à choisir le ou les éventuels composés à ajouter aux compositions moussantes et émulsions selon l'invention de manière telle que les propriétés avantageuses attachées intrinsèquement à ces compositions ne soient pas, ou substantiellement pas, altérées par l'addition envisagée.

Par chargés, il faut comprendre des particules incolores ou blanches, minérales ou de synthèse, lamellaires ou non lamellaires, destinées à donner du corps ou de la rigidité à la composition et/ou de la douceur, de la matité et de l'uniformité au maquillage. Comme charges, on peut notamment citer le talc, le mica, la silice, le nitrure de bore, l'oxychlorure de bismuth, le kaolin, les poudres de Nylon telles que Nylon-12 (Orgasol commercialisé par la société Atochem), les poudres de polyéthylène, le Téflon (poudres de polymères de tétrafluoroéthylène), les poudres de polyuréthanne, les poudres de polystyrène, les poudres de polyester, l'amidon éventuellement modifié, des microsphères de copolymères, telles que celles commercialisées sous les dénominations Expancel par la société Nobel Industrie, les microéponges comme le Polytrap commercialisé par la société Dow Corning, les microbilles de résine de silicone telles que celles commercialisées par la société Toshiba sous la dénomination Tospearl, le carbonate de calcium précipité, le carbonate et l'hydrocarbonate de magnésium, l'hydroxyapatite, les microsphères de silice creuses (Silica Beads de la Société Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.

Par pigments, il faut comprendre des particules blanches ou colorées, minérales ou organiques, insolubles dans le milieu, destinées à colorer et/ou opacifier la composition. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes (nanopigments de dioxyde de titane), les nanozincs (nanopigments d'oxyde de zinc), le bleu ferrique. Parmi les pigments organiques, on peut citer le noir de carbone, et les laques comme les sels de calcium, de baryum, d'aluminium ou de zirconium, de colorants acides tels que les colorants halogéno-acides, azoïques ou anthraquinoniques.

Par nacres, il faut comprendre des particules irisées qui réfléchissent la lumière. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Dans le cas des émulsions, on peut en particulier ajouter à celles-ci un agent gélifiant afin de régler la texture de l'émulsion et d'accéder à une large gamme de textures allant du lait à la crème.

Les gélifiants utilisables peuvent être des gélifiants hydrophiles. A titre d'exemples de gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles.

Les compositions moussantes et émulsions de l'invention peuvent se présenter notamment sous la forme d'une composition cosmétique, de maquillage, de nettoyage ou de soin, susceptible d'être appliquée sur la peau y compris le cuir chevelu, les ongles, les cheveux, les cils, les sourcils, les yeux, les muqueuses et les semi-muqueuses, et toute autre zone cutanée du corps et du visage.

Comme produit de nettoyage et/ou de démaquillage de la peau (du corps ou du visage), les compositions moussantes selon l'invention peuvent être utilisées de deux façons :
- la première utilisation consiste à étaler le gel dans les mains, à l'appliquer sur le visage ou sur le corps puis à le masser en présence d'eau pour développer la mousse directement sur le visage ou le corps,
- l'autre utilisation possible de ce type de produit consiste à développer la mousse dans les paumes des mains avant d'être appliquée sur le visage ou le corps.

Si la composition est suffisamment fluide, elle peut être conditionnée en flacon air spray ou aérosol automoussant. Le produit est alors délivré sous forme de mousse qui s'applique directement sur la peau ou les cheveux.

Dans tous les cas, la mousse est ensuite rincée.

Un autre objet de l'invention consiste en un procédé cosmétique de nettoyage et/ou de démaquillage de la peau, du cuir chevelu et/ou des cheveux, caractérisé par le fait qu'on applique la composition de l'invention, sur la peau, sur le cuir chevelu et/ou sur les cheveux, en présence d'eau, et qu'on élimine la mousse formée et les résidus de salissure par rinçage à l'eau.

Les émulsions selon l'invention peuvent être utilisées dans beaucoup d'applications cosmétiques ou dermatologiques, notamment pour le traitement, le soin et/ou le maquillage de la peau du visage et/ou du corps, des muqueuses (lèvres), du cuir chevelu et/ou des fibres kératiniques (cheveux ou cils), et/ou pour protéger la peau des rayons U.V.

Aussi, la présente invention a pour objet l'utilisation cosmétique de l'émulsion cosmétique telle que définie ci-dessus, pour le traitement, le soin, la protection et/ou le maquillage de la peau du visage et/ou du corps, des muqueuses, du cuir chevelu et/ou des fibres kératiniques.

D'autres caractéristiques et avantages de l'invention apparaîtront mieux à la lecture de la description qui suit donnée à titre illustratif et non limitatif.

### Description détaillée des modes de réalisation

Les exemples suivants illustrent l'utilisation de polymères comportant des unités hydrosolubles et des unités à LCST pour la réalisation de compositions moussantes et d'émulsions.

Les polymères utilisés dans ces exemples sont constitués par un squelette d'acide polyacrylique PAA) portant des chaînes latérales ou greffons constituées par des unités à LCST. Ils sont caractérisés par la masse molaire du squelette hydrosoluble (acide polyacrylique), la nature chimique des chaînes à LCST, leur proportion massique dans le polymère et leur masse molaire.

Les caractéristiques des polymères utilisés sont données dans le tableau 1.

**Tableau 1**

| | Squelette hydrosoluble | Greffons (unités à LCST) | Proportion : unités à LCST dans polymère final (en poids) | Taux de greffage (% en mole) |
|---|---|---|---|---|
| Polymère 1 | Acide polyacrylique; PM=450000 | (OE)₆(OP)₃₉ statistique Jeffamine M-2005; PM=2600 | 51 % | 3,9% |
| Polymère 2 | Acide polyacrylique; PM=550000 | PolyN-isopropylacrylamide (pNIPAM) PM= 10 000 | 49 % | 0,9% |
| Polymère 3 | Acide polyacrylique; PM=750000 | (OE)₆(OP)₃₉ statistique Jeffamine M-2005; PM=2600 | 45% | 3,1% |

Ces polymères sont préparés de la façon suivante.

### Préparation du Polymère 1

Dans un réacteur de 500 ml muni d'un réfrigérant, on dissout 3 grammes d'acide polyacrylique de masse molaire moyenne 450 000 g/mole (Aldrich) dans 220 ml de N-méthyl pyrrolidone sous agitation à 60°C pendant 12 heures.

On dissout 4,181 grammes de copolymère (OE)₆(OP)₃₉ statistique mono-aminé, de masse molaire 2600 g/mole ayant un point de trouble, à une concentration de 1% en poids dans l'eau, de 16°C (Jeffamine M-2005 de Huntsman) dans 50 ml de N-méthylpyrrolidone sous agitation, à 20°C, pendant 15 minutes. La solution obtenue est ajoutée goutte à goutte au milieu réactionnel contenant l'acide polyacrylique, sous vive agitation à 60°C.

On dissout 2,158 grammes de dicyclohexylcarbodiimide dans 30 ml de N-méthylpyrrolidone sous agitation à 20°C pendant 15 minutes. La solution obtenue est ajoutée goutte à goutte au milieu réactionnel contenant l'acide polyacrylique et le copolymère (OE)₆(OP)₃₉ statistique mono-aminé, sous vive agitations à 60°C. On agite le mélange final pendant 12 heures à 60°C.

On refroidit le mélange à 20°C puis on le place dans un réfrigérateur à 4°C pendant 24 heures. Les cristaux de dicyclohexylurée formés sont éliminés par filtration du milieu réactionnel.

Le polymère est alors neutralisé à l'aide de 19 g de soude à 35% (4 fois en excès par rapport au nombre de mole d'acide acrylique), ce qui conduit à sa précipitation. Après 12 heures au repos, le milieu réactionnel est filtré afin de récupérer le polymère précipité. Celui-ci est séché sous vide à 35°C pendant 24 heures.

On récupère 13,55 grammes de solide qui sont dissous dans 2 litres d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.

On obtient 7,05 grammes d'acide polyacrylique (450 000 g/mole) greffé par 3,9% (en mole) de copolymère (OE)₆(OP)₃₉ statistique mono-aminé.

La proportion massiques des unités à LCST dans le polymère final est de 51%.

Le polymère ainsi obtenu présente une solubilité dans l'eau, à 20°C, d'au moins 10 g/l.

### Préparation du polymère 2

Le polymère 2 qui comporte des greffons de poly-N-isopropylacrylamide (pNIPAM) est préparé par un procédé en 2 étapes :

### 1) Synthèse d'oligomères pNIPAM portant une extrémité réactive aminée

Dans un ballon tricol de 250 ml muni d'un réfrigérant et d'une arrivée d'azote sont introduits 8 grammes de N-isopropylacrylamideo et 80 ml de diméthylsulfoxyde. Ce mélange est chauffé sous agitation à 29°C à l'aide d'un bain Marie et placé sous un barbotage d'azote. Après 45 minutes, 0,161 gramme de chlorhydrate d'aminoéthanethiol préalablement dissous dans 4 ml de diméthylsulfoxyde sont ajoutés au milieu réactionnel. 5 minutes après, 0,191 gramme de persulfate de potassium dissous dans 8 ml de diméthylsulfoxyde sont ajoutés au milieu réactionnel. Ce milieu réactionnel est maintenu sous agitation et atmosphère d'azote pendant 3 heures à 29°C.

Les oligomères de poly N-isopropylacrylamide (pNIPAM) synthétisés sont isolés par précipitation du milieu réactionnel dans un mélange d'acétone (40% en volume) et d'hexane (60%).

### 2) Greffage des oligomères de pNIPAM sur de l'acide polyacrylique

Dans un ballon tricol de 250 ml muni d'un réfrigérant sont dissous dans 100 ml de 1-méthyl 2-pyrrolydone 3 grammes d'acide polyacrylique de masse molaire 550 000 g/mole, sous agitation à 60°C pendant 12 heures. 3,757 grammes d'oligomères pNIPAM préalablement dissous dans 25 ml de 1-méthyl 2-pyrrolidone sont introduits goutte à goutte dans le milieu réactionnel sous agitation. 15 minutes après, 0,776 gramme de dicyclohexylcarbodiimide préalablement dissous dans 25 ml de 1-méthyl 2-pyrrolidone sont introduits goutte à goutte dans le milieu réactionnel sous vive agitation. Le milieu réactionnel est maintenu pendant 12 heures à 60°C sous agitation.

Le milieu réactionnel est alors refroidi à 20°C puis placé dans un réfrigérateur à 4°C pendant 24 heures. Les cristaux de dicyclohexylurée formés sont alors éliminés par filtration. Le polymère est alors neutralisé à l'aide de 19 g de soude à 35% (4 fois en excès par rapport au nombre de mole d'acide acrylique), ce qui conduit à sa précipitation. Après 12 heures au repos, le milieu réactionnel est filtré afin de récupérer le polymère précipité. Celui-ci est séché sous vide à 35°C pendant 24 heures.

On récupère 10,2 grammes de solide qui sont dissous dans 2 litres d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.

On obtient 4,8 grammes d'acide polyacrylique (550 000 g/mole) greffé par 0,9% (en mole) de poly N-isopropylacrylamide.

La proportion massique des unités à LCST dans le polymère final est de 49%.

### Préparation du polymère 3

Dans un réacteur de 1 litre muni d'un réfrigérant, on dissout 1,51 grammes d'acide polyacrylique de masse molaire moyenne 750 000 g/mole (Aldrich) dans 350 ml d'eau désionisée sous agitation à 20°C pendant 12 heures. Le pH du milieu réactionnel est alors ajusté à 8 à l'aide d'une solution de soude 1M.

On dissout 1,60 grammes de copolymère (OE)₆ (OP)₃₉ statistique mono-aminé (Jeffamine M-2005 de Huntsman) dans 100 ml d'eau désionisée sous agitation à 5°C. pendant 30 minutes. La solution obtenue est ajoutée goutte à goutte au milieu réactionnel sous vive agitation.

On dissout 1,84 grammes de chlorhydrate de 1-(3-diméthylaminopropyl)-3-éthyl carbodiimide dans 50 ml d'eau désionisée sous agitation à 20°C pendant 15 minutes. La solution obtenue est ajoutée sous vive agitation, goutte à goutte, au milieu réactionnel précédent qui est alors chauffé à 60°C pendant 6 heures.

Le milieu réactionnel est refroidi à 20°C puis concentré et précipité dans l'acétone. Le polymère sous forme solide est récupéré par filtration et lavé avec un excès d'acétone. La poudre est broyée et séchée sous vide à 35°C.

On obtient 3,33 g d'acide polyacrylique (750 000 g/mole) greffé par 3,1% (en mole) de copolymère (OE)₆(OP)₃₉ statistique mono-aminé (rendement 94%).

On détermine les températures de démixtion des unités à LCST des polymères, soit, des unités Jeffamine et des unités pNIPAM.

Ces températures de démixtion sont déterminées par spectroscopie UV visible en mesurant, à une longueur d'onde égale à 500 nm, la transmittance de solutions aqueuses de ces unités en fonction de la température ; la température de démixtion est identifiée à la température au delà de laquelle la transmittance devient inférieure de 10% à sa valeur à 10°C. Les résultats obtenus pour diverses concentrations massiques, sont regroupés dans le tableau 2 suivant :

**Tableau 2**

| Concentration massique en solution aqueuse (%) | (OE)₆(OP)₃₉ statistique Jeffamine M-2005; PM=2600 | PolyN-isopropylacrylamide pNIPAM PM= 10 000 |
|---|---|---|
| 0,025 | 374°C | 37°C |
| 0,05 | 36°C | 36°C |
| 0,15 | / | 32°C |
| 1 | 16°C | 32°C |

Dans les exemples ci-dessus, les mesures de viscosité ont été réalisées à l'aide d'un rhéomètre Haake RS150 équipé d'une géométrie cône/plan (35 mm, 2°) et d'un bain thermostaté afin de contrôler la température entre 5 et 80°C. Les mesures ont été effectuées dans le mode écoulement, à une vitesse de cisaillement imposée égale à 10 s⁻¹, en faisant varier la température de 20 à 40°C à la vitesse de 0,5°C/min.

### Exemple 1 : Activité superficielle des polymères 1 et 2 comportant un squelette hydrosoluble et des greffons constitués par des unités à LCST

Les mesures de tensions superficielles (σ) ont été effectuées à l'aide d'un tensiomètre à anneau (Tensiomètre K12 de Krüss), équipé d'un bain thermostaté permettant d'ajuster la température entre 5°C et 80°C. Les solutions de polymères ont été préparées par simple dissolution sous agitation de la quantité adéquate de polymère à l'état de poudre dans l'eau, à la température ambiante, pour obtenir une concentration massique en polymère de 0,05 ou 0,1%.

Les mesures ont été faites à deux températures :
- 15°C, valeur inférieure à la température de démixtion des greffons à LCST (Polymères 1 et 2) aux concentrations de 0,05 et 0,1%,
- 38°C, valeur supérieure à la température de démixtion des greffons à LCST (Polymères 1 et 2) à ces concentrations.

Les résultats sont donnés dans le tableau 3.

**Tableau 3**

| | Eau | Polymère 1 | Polymère 2 | Squelette PAA | Greffons du Polymère 1 | Greffons du Polymère 2 |
|---|---|---|---|---|---|---|
| σ (eau) mN/m à 15°C | 73,5 | / | / | / | / | / |
| σ (eau) mN/m à 38°C | 69,5 | / | / | / | / | / |
| σ (0,05%) mN/m à 15 °C | / | 45 | 44 | 55 | 41 | 35 |
| σ(0,05%) mN/m à 38°C | / | 36 | 38 | 51 | Non mesurable car insoluble | Non mesurable car insoluble |
| σ (0,1%) mN/m à 15°C | / | 45 | 42 | / | / | / |
| σ (0,1%) mN/m à 38°C | / | 35 | 36 | / | / | / |

Les concentrations sont données en pourcentages massiques.

Les résultats du tableau 3 montrent que l'on obtient un abaissement important de la tension superficielle de l'eau avec les polymères 1 et 2, celui-ci étant encore plus important au-dessus de la température de démixtion.

### Exemple 2 : Formule moussante composée du Polymère 3 à une concentration massique de 0,3%, T = 25°C.

On prépare la mousse à partir d'une solution aqueuse du polymère 3 à 0,3% en masse (5 grammes de solution dans des pilluliers de 10 ml) soumise à une agitation à l'aide d'un appareil DIAX 600 (Heidolph) pendant 5 minutes à 8000 RPM, puis pendant 1 minute à 13500 RPM. L'axe utilisé a un diamètre extérieur de 10 mm (référence 10F).

A titre comparatif, on prépare des mousses de la même façon mais en utilisant uniquement a) le squelette hydrosoluble du polymère 3 dans l'eau, à une concentration massique de 0,15%, et b) les greffons à LCST dans l'eau à une concentration massique de 0,15%.

L'évolution de l'aspect macroscopique des mousses ainsi obtenues est suivie dans le temps ; le pouvoir moussant est d'autant plus grand que la hauteur de mousse est importante dans le récipient.

Dans le cas du polymère 3 à une concentration massique de 0,3%, on obtient une mousse importante qui n'évolue pratiquement pas après 1 heure ; elle se déstabilise légèrement au bout de 3 jours.

Dans le cas du squelette hydrosoluble du polymère 3, à une concentration massique de 0,15%, la production de mousse est très faible, la mousse se déstabilise au bout de 30 minutes et disparaît complètement au bout de 3 jours. Dans le cas des greffons du polymère 3 à une concentration massique de 0,15%, on obtient une mousse qui disparaît complétement au bout de 15 minutes.

Ainsi, le polymère 3 présente des propriétés moussantes supérieures à celles du squelette hydrosoluble ainsi qu'à celles des greffons à LCST. Les solutions aqueuses de Polymère 3 (à 0,3%) et de squelette PAA (à 0,15%) ayant la même viscosité (≈ 0,5 Pa.s pour une vitesse de cisaillement de 10 s⁻¹), la meilleure stabilité de la mousse obtenue avec le Polymère 3 par rapport celle contenant le squelette hydrosoluble n'est pas due à un effet d'épaississement.

### Exemple 3 : Formule moussante composée du Polymère 2 à une concentration massique de 0,1%, T = 15°C et 38°C.

On prépare des mousses à partir d'une solution aqueuse du polymère 2 à 0,1% en masse (5 grammes de solution dans des pilluliers de 10 ml) soumise à une agitation à l'aide d'un appareil DIAX 600 (Heidolph) pendant 5 minutes à 8000 RPM, puis pendant 1 minute à 13500 RPM. L'axe utilisé a un diamètre extérieur de 10 mm (référence 10F).

On prépare de la même façon des mousses à partir d'une solution aqueuse comprenant 0,15% en masse du squelette hydrosoluble du polymère 2 et d'une solution aqueuse des greffons à LCST du polymère 2, à une concentration massique de 0,15%.

On suit dans le temps l'évolution de l'aspect macroscopique des mousses ainsi obtenues, à une température de 15°C et de 38°C, dans le cas du polymère 2 et à une température de 25°C dans le cas du squelette hydrosoluble et des greffons à LCST.

Dans le cas du polymère 2, on obtient à 15°C une mousse importante mais la hauteur de mousse diminue au bout de 30 minutes (50% de mousse en moins) et elle disparaît au bout de 2 heures. A 38°C, on obtient également une mousse importante qui persiste 2 heures après (il reste 40% de mousse) et qui est toujours partiellement présente après 22 heures (25% de mousse reste encore).

Dans le cas du squelette hydrosoluble du polymère 2, on obtient une très faible hauteur de mousse à 25°C et la mousse disparaît au bout de 5 minutes. Dans le cas des greffons à LCST, on obtient une mousse importante à 25°C, qui se déstabilise instantanément (80% de mousse et 20% de liquide) puis de façon importante au bout de 30 minutes (seulement 15% de mousse reste) et jusqu'à disparaître après 2 heures.

Ainsi, le polymère 2 présente des propriétés moussantes supérieures à celle du squelette hydrosoluble et des greffons à LCST. Ce pouvoir moussant est observé sur une large gamme de températures et est amélioré lorsque la température devient supérieure à la température de démixtion des chaînes à LCST qui est de 36°C, à la concentration massique de 0,15%. Cette amélioration du pouvoir moussant du polymère 2 de 15°C à 38°C n'est pas liée à un effet de gélification en température car les viscosités du polymère 2 en solution aqueuse à 0,1% sont les suivantes :
- viscosité à 15°C (10 s⁻¹) : 0,007 Pa.s ;
- viscosité à 38°C (10 s⁻¹) : 0,005 Pa.s.

### Exemple 4 : Activité interfaciale eau/huile des polymères 1 et 2 comprenant un squelette hydrosoluble et des greffons à LCST

Les mesures de tensions interfaciales (σ) sont effectuées à l'aide d'un tensiomètre à profil de goutte (Tensiomètre G10 de Krüss), équipé d'un bain thermostaté permettant d'ajuster la température entre 5°C et 80°C. Les solutions de polymères ont été préparées par simple dissolution sous agitation de la quantité adéquate de polymère à l'état de poudre dans l'eau, à la température ambiante pour obtenir des concentrations massiques en polymère de 0,05% ou 0,1%. Les mesures ont été effectuées à une interface eau/huile de Parléam, à deux températures :
- 15°C, valeur inférieure à la température de démixtion des greffons à LCST (Polymères 1 et 2),
- 38°C, valeur supérieure à la température de démixtion des greffons à LCST (Polymères 1 et 2).

Les résultats sont donnés dans le tableau 4.

**Tableau 4**

| | Eau | Polymère 1 | Polymère 2 | Squelette PAA | Greffons du Polymère 1 | Greffons du Polymère 2 |
|---|---|---|---|---|---|---|
| σ (eau) mN/m à 15°C | 40 | / | / | / | / | / |
| σ (eau) mN/m à 38°C | 39 | / | / | / | / | / |
| σ (0,05%) mN/m à 15°C | / | / | / | 39 | 9 | 8 |
| σ (0,05%) mN/m à 38°C | / | / | / | 38 | Non mesurable car insoluble | Non mesurable car insoluble |
| σ (0,1 %) mN/m à 15°C | / | 15 | 8 | / | / | / |
| σ (0,1%) mN/m à 38°C | / | 10 | 15 | / | / | / |

Les concentrations sont données en pourcentages massiques dans la phase aqueuse.

Les résultats du tableau 4 montrent que la tension interfaciale de l'eau en présence des polymères 1 et 2 est abaissée de 25 ou 22 mM/m à 15°C et de 29 à 24 mM/m à 38°C dans le cas de solutions des polymères 1 et 2 à 0,1% en masse.

### Exemple 5 : Emulsion contenant le Polymère 3 à une concentration massique dans la phase aqueuse de 0,3%, T = 25°C.

On prépare l'émulsion, à partir d'une solution aqueuse du polymère 3 à 0,3% en masse (4,8 g) et d'huile de Parléam (1,2 g), et on soumet le mélange à une agitation à l'aide d'un appareil DIAX 600 (Heidolph) pendant 5 minutes à 8000 RPM, puis pendant 1 minute à 13500 RPM. L'axe utilisé a un diamètre extérieur de 10 min (référence F10). L'émulsification est réalisée dans des pilluliers de volume 10 ml. La fraction massique en huile de Parléam a été fixée à 20%.

On prépare de la même façon des émulsions à partir d'une solution aqueuse du squelette hydrosoluble du polymère 3 à une concentration massique de 0,15%, et d'une solution aqueuse des greffons à LCST du polymère 3, à une concentration massique de 0,15%.

L'évolution de l'aspect macroscopique des émulsions est suivie dans le temps ; le pouvoir émulsionnant est d'autant plus grand que la hauteur de la phase émulsionnée est importante.

Dans le cas du polymère 3 à 0,3% en masse, à 25°C, l'ensemble de la composition est émulsionné et reste stable pendant 64 jours. Dans le cas du squelette hydrosoluble du polymère 3 à 0,15% en masse, on obtient une bonne hauteur d'émulsion mais celle-ci diminue après 64 jours.

Dans le cas des greffons à LCST du polymère 3, on obtient une hauteur d'émulsion plus faible et l'émulsion disparaît au bout de 52 heures.

Ainsi, le polymère 3 présente des propriétés émulsionnantes supérieures à celles du squelette hydrosoluble et des greffons à LCST. Les solutions aqueuses de Polymère 3 (à 0,3%) et de squelette PAA (à 0,15%) ayant la même viscosité (≈ 0,5 Pa.s pour une vitesse de cisaillement de 10 s⁻¹), la meilleure stabilité de l'émulsion obtenue avec le polymère 3 par rapport celle contenant le squelette hydrosoluble n'est pas due à un effet d'épaississement.

### Exemple 6 : Emulsions contenant le Polymère 2, à une concentration dans la phase aqueuse de 0,3% (en masse), T = 15°C et 38°C.

On prépare les émulsions à partir d'une solution aqueuse du polymère 3 à 0,3% en masse (4,8 g) et d'huile de Parléam (1,2 g) ; on soumet le mélange à une agitation à l'aide d'un appareil DIAX 600 (Heidolph) pendant 5 minutes à 8000 RPM, puis pendant 1 minute à 13500 RPM. L'axe utilisé a un diamètre extérieur de 10 mm (Référence 10F). L'émulsification est réalisée dans des pilluliers de volume 10 ml. La fraction massique en huile de Parléam a été fixée à 20%.

L'évolution de l'aspect macroscopique des émulsions est suivie dans le temps ; le pouvoir émulsionnant est d'autant plus grand que la hauteur de la phase émulsionnée est importante.

A 15°C, l'ensemble du volume est initialement émulsionné pour le polymère 2 et la hauteur de la phase émulsionnée disparaît de moitié après 63 jours.

A 38°C, l'émulsion obtenue avec le polymère 2 est également très importante, et elle reste stable pendant. 63 jours.

Ainsi, le polymère 2 présente des propriétés d'émulsification sur une large gamme de températures et son pouvoir émulsionnant est amélioré lorsque la température devient supérieure à la température de démixtion des chaînes à LCST (32°C pour une concentration de 0,3%). Cette amélioration n'est pas liée à un effet de gélification en température car les viscosités du polymère 2 en solution aqueuse à 0,3% sont les suivante
- viscosité à 15°C (10 s⁻¹) : 0,12 Pa.s,
- viscosité à 38°C (10 s⁻¹) : 0,12 Pa.s.

On décrit ci-après des exemples de compositions cosmétiques sous forme de mousse ou d'émulsion.

### Exemple 7 : Composition moussante fluide

| | |
|---|---|
| Glycérine | 5 % en poids |
| Polymère 3 | 0,3 % en poids |
| Conservateur | 0,4 % en poids |
| Ethylène diamine tétraacétate de sodium | 0,2 % en poids |
| Eau déminéralisée | 94,1 % en poids |

Cette composition moussante est obtenue par dissolution du polymère 3 sous forme de poudre dans l'eau déminéralisée sous agitation à la température ambiante pendant 3 heures ; les autres constituants sont alors introduits dans cette solution et l'agitation est maintenue pendant 30 minutes.

La formule obtenue est une composition moussante fluide pouvant être utilisée de 5°C à 60°C.

### Exemple 8 : Lait pour le corps

Ce lait contient une phase huileuse et une phase aqueuse ayant les compositions suivantes :

| | |
|---|---|
| Phase huileuse | |
| Huile de Parléam | 9% en poids du lait |
| Cyclodiméthylsiloxane | 6% en poids du lait |

| | |
|---|---|
| Phase aqueuse | |
| Polymère 3 | 0,3% en poids du lait |
| Conservateur | 0,2% en poids du lait |
| Eau déminéralisée | 84,5% en poids du lait |

La phase aqueuse est obtenue par dissolution du Polymère 3 sous forme de poudre dans l'eau déminéralisée et le conservateur sous agitation pendant 3 heures. La phase huile est alors introduite lentement dans la phase aqueuse sous agitation à l'aide d'un mélangeur de type Moritz à la vitesse de 4000 RPM pendant 20 minutes.

La formule obtenue est une émulsion ayant une belle texture fluide et aqueuse.

### REFERENCES

**[1]** D. HOURDET et al., Polymer, 1994, vol. 35, n°12, pages 2624-2630;
**[2]** F. L'ALLORET et al., Coll. Polym. Sci., 1995, vol. 273, n°12, pages 1163-1173,;
**[3]** F. L'ALLORET, Revue de l'Institut Français du Pétrole, 1997, vol. 52, n°2, pages 117-128;
**[4]** EP-A-0 583 814 ;
**[5]** EP-A-0 629 649 ;
**[6]** WO-A-95/24430 ;
**[7]** US-A-5, 939, 485,
**[8]** WO-A-97/00275,
**[9]** WO-A-98/48768,
**[10]** WO-A-00/35961
**[11]** EP-A-566 438
**[12]** FR-A-2 739 556

## Revendications

1. Utilisation d'un polymère comprenant des unités hydrosolubles et des unités à Lower Critical Solution Temperature (LCST), les unités à LCST présentant dans l'eau une température de démixtion de 5 à 40°C à une concentration massique de 1% dans l'eau, pour abaisser la tension superficielle ou la tension interfaciale de l'eau.

2. Utilisation selon la revendication 1, dans laquelle l'abaissement de la tension superficielle ou de la tension interfaciale de l'eau est d'au moins 15 mN/m pour une concentration massique en polymère dans l'eau de 0,1% dans la gamme de température allant de 5 à 80°C.

3. Utilisation selon la revendication 1, dans laquelle l'abaissement de la tension superficielle ou de la tension interfaciale de l'eau est d'au moins 20 mN/m pour une concentration massique en polymère dans l'eau de 0,1% lorsque la température est supérieure à la température de démixtion des unités à LCST à cette concentration.

4. Utilisation d'un polymère comprenant des unités hydrosolubles et des unités à LCST, les unités à LCST présentant dans l'eau une température de démixtion de 5 à 40°C à une concentration massique de 1% dans l'eau, pour fabriquer une mousse.

5. Utilisation d'un polymère comprenant des unités hydrosolubles et des unités à LCST, les unités à LCST présentant dans l'eau une température de démixtion de 5 à 40°C à une concentration massique de 1% dans l'eau, pour fabriquer une mousse comprenant de plus un tensioactif moussant à une concentration massique égale ou inférieure à 5%.

6. Utilisation d'un polymère comprenant des unités hydrosolubles et des unités à LCST, les unités à LCST présentant dans l'eau une température de démixtion de 5 à 40°C à une concentration massique de 1% dans l'eau, pour fabriquer une émulsion sans tensioactif émulsionnant additionnel ou contenant un tensioactif émulsionnant additionnel à une concentration massique égale ou inférieure à 1%.

7. Composition moussante comprenant une phase aqueuse contenant un polymère comprenant des unités hydrosolubles et des unités à LCST, les unités à LCST présentant dans l'eau une température de démixtion de 5 à 40°C à une concentration massique de 1% dans l'eau.

8. Composition moussante selon la revendication 7, dans laquelle le polymère se présente sous la forme d'un polymère séquencé (ou blocs) comprenant des unités hydrosolubles alternées avec des unités à LCST, ou sous la forme d'un polymère greffé dont le squelette est formé d'unités hydrosolubles et est porteur de greffons constitués d'unités à LCST, cette structure pouvant être partiellement réticulée, ou encore sous la forme d'un polymère greffé dont le squelette est formé d'unités à LCST et est porteur de greffons constitués d'unités hydrosolubles, cette structure pouvant être partiellement réticulée.

9. Composition moussante selon l'une quelconque des revendications 7 et 8, dans laquelle les unités hydrosolubles sont obtenues par polymérisation radicalaire d'au moins un monomère choisi parmi :
- l'acide (méth)acrylique ;
- les monomères vinyliques de formule (I) suivante : 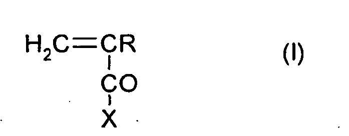
dans laquelle :
- R est choisi parmi H, -CH₃, -C₂H₅ ou -C₃H₇, et
- X est choisi parmi :
- les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor); un groupement sulfonique (-SO₃⁻) , sulfate- (-SO₄⁻) , phosphate (-PO₄H₂) ; hydroxy (-OH) ; amine primaire (-NH₂); amine secondaire (-NHR₁), tertiaire (-NR₁R₂) ou quaternaire (-N⁺R₁R₂R) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
- les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂) ; amine primaire (-NH₂) ; amine secondaire (NHR₁) , tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
- l'anhydride maléique ;
- l'acide itaconique ;
- l'alcool vinylique de formule CH₂=CHOH ;
- l'acétate de vinyle de formule CH₂=CH-OCOCH₃ ;
- les N-vinyllactames tels que la N-vinylpyrrolidone, la N-vinylcaprolactame et la N-butyrolactame ;
- les vinyléthers de formule CH₂=CHOR₆ dans laquelle R₆ est un radical hydrocarboné, linéaire ou ramifié, saturé ou insaturé, ayant de 1 à 6 atomes de carbone ;
- les dérivés hydrosolubles du styrène, notamment le styrène sulfonate ;
- le chlorure de diméthyldiallyl ammonium ; et
- la vinylacétamide.

10. Composition moussante selon l'une quelconque des revendications 7 et 8, dans laquelle les unités hydrosolubles sont constituées en tout ou en partie d'un ou plusieurs des composants suivants :
- les polyuréthannes hydrosolubles,
- la gomme de xanthane,
- les alginates et leurs dérivés tels que l'alginate de propylèneglycol,
- les dérivés de cellulose et notamment la carboxyméthylcellulose, l'hydroxypropylcellulose, l'hydroxyéthylcellulose et l'hydroxyéthylcellulose quaternisée,
- les galactomannanes et leurs dérivés tels que la gomme Konjac, la gomme de guar, l'hydroxypropylguar, l'hydroxypropylguar modifié par des groupements méthylcarboxylate de sodium, et le chlorure de guar hydroxypropyl triméthyl ammonium, et
- la polyéthylène-imine.

11. Composition moussante selon l'une quelconque des revendications 7 à 10, dans laquelle les unités hydrosolubles ont une masse molaire allant de 1 000 g/mole à 5 000 000 g/mole lorsqu'elles constituent le squelette hydrosoluble d'un polymère greffé, ou une masse molaire allant de 500 g/mole à 100 000 g/mole lorsqu'elles constituent un bloc d'un polymère multiblocs ou lorsqu'elles constituent les greffons d'un polymère greffé.

12. Composition moussante selon l'une quelconque des revendications 7 à 11, dans laquelle les unités à LCST sont constituées d'un ou plusieurs des polymères suivants :
- les polyéthers tels que le polyoxyde d'éthylène (POE), le polyoxyde de propylène (POP), et les copolymères statistiques d'oxyde d'éthylène (OE) et d'oxyde de propylène (OP),
- les polyvinylméthyléthers,
- les dérivés polymériques N-substitués de l'acrylamide tels que le poly N-isopropyl acrylamide, le poly N-éthyl acrylamide et les copolymères de N-isopropyl acrylamide ou de N-éthyl acrylamide et d'un monomère vinylique répondant à la formule (I) donnée dans la revendication 9, ou d'un monomère choisi parmi l'anhydride maléique, l'acide itaconique, la vinylpyrrolidone, le styrène et ses dérivés, le chlorure de diméthyldiallyl ammonium, la vinylacétamide, les vinyléthers et les dérivés de l'acétate de vinyle ; et
- le polyvinylcaprolactame et les copolymères de vinyl caprolactame et d'un monomère vinylique répondant à la formule (I) donnée dans la revendications 9, ou d'un monomère choisi parmi l'anhydride maléique, l'acide itaconique, la vinylpyrrolidone, le styrène et ses dérivés, le chlorure de diméthyldiallyl ammonium, la vinylacétamide, les vinyléthers et les dérivés de l'acétate de vinyle.

13. Composition moussante selon l'une quelconque des revendications 7 à 11, dans laquelle les unités à LCST sont constituées de poly oxydes de propylène de formule (POP)ₙ avec n étant un nombre entier de 10 à 50, ou copolymères statistiques d'oxyde d'éthylène (OE) et d'oxyde de propylène (OP), représentés par la formule :
(OE)ₘ (OP)ₙ
dans laquelle m est un nombre entier allant de 1 à 40, de préférence de 2 à 20, et n est un nombre entier allant de 10 à 60, de préférence de 20 à 50.

14. Composition moussante selon la revendication 13, dans laquelle la masse molaire des unités à LCST est de 500 à 5 300 g/mole, de préférence de 1 500 à 4 000 g/mole.

15. Composition moussante selon la revendication 12, dans laquelle les unités à LCST sont constituées par le poly-N-isopropylamide ou le poly-N-éthylacrylamide ou un copolymère de N-isopropylacrylamide ou de N-éthylacrylamide et d'un monomère répondant à la formule (I) donnée dans la revendication 9, ou d'un monomère choisi parmi l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

16. Composition moussante selon la revendication 15, dans laquelle la masse molaire des unités à LCST est de 1 000 g/mole à 500 000 g/mole, de préférence de 2 000 à 50 000 g/mole.

17. Composition moussante selon l'une quelconque des revendications 7 à 11, dans laquelle les unités à LCST sont constituées par un polyvinylcaprolactame ou un copolymère de vinylcaprolactame et d'un monomère vinylique répondant à la formule (I) donnée dans la revendication 9, ou d'un monomère choisi parmi l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

18. Composition moussante selon la revendication 17, dans laquelle la masse molaire des unités à LCST est de 1 000 à 500 000 g/mole, de préférence de 2 000 à 50 000 g/mole.

19. Composition moussante selon l'une quelconque des revendications 7 à 18, dans laquelle la proportion massique des unités à LCST du polymère est de 5 à 70%, de préférence de 20 à 65% et mieux encore de 30 à 60%, par rapport au polymère.

20. Composition moussante selon l'une quelconque des revendications 7 à 19, dans laquelle la température de démixtion des unités à LCST est de 5 à 40°C, pour une concentration massique des unités à LCST dans l'eau de 1%.

21. Composition moussante selon l'une quelconque des revendications 7 à 20, dans laquelle la concentration massique en polymère de la phase aqueuse est égale ou inférieure à 5%, de préférence de 0,01 à 5%.

22. Composition moussante selon l'une quelconque des revendications 7 à 21, dans laquelle la phase aqueuse comprend en outre un tensioactif moussant à une concentration massique ne dépassant pas 5%.

23. Emulsion huile-dans-eau comprenant une phase aqueuse et une phase huileuse dispersée dans la phase aqueuse, dans laquelle la phase aqueuse comprend un polymère comprenant des unités hydrosolubles et des unités à LCST, les unités à LCST présentant dans l'eau une température de démixtion de 5 à 40°C à une concentration massique de 1% dans l'eau.

24. Emulsion eau-dans huile-dans eau comprenant une émulsion eau-dans huile dispersée dans une phase aqueuse externe, dans laquelle la phase aqueuse externe comprend un polymère comprenant des unités hydrosolubles et des unités à LCST, les unités à LCST présentant dans l'eau une température de démixtion de 5 à 40°C à une concentration massique de 1% dans l'eau.

25. Emulsion selon la revendication 23 ou 24, dans laquelle le polymère se présente sous la forme d'un polymère séquencé (ou blocs) comprenant des unités hydrosolubles alternées avec des unités à LCST, ou sous la forme d'un polymère greffé dont le squelette est formé d'unités hydrosolubles et est porteur de greffons constitués d'unités à LCST, cette structure pouvant être partiellement réticulée, ou encore sous la forme d'un polymère greffé dont le squelette est formé d'unités à LCST et est porteur de greffons constitués d'unités hydrosolubles, cette structure pouvant être partiellement réticulée.

26. Emulsion selon l'une quelconque des revendications 23 à 25, dans laquelle les unités hydrosolubles sont obtenues par polymérisation radicalaire d'au moins un monomère choisi parmi :
- l'acide (méth)acrylique ;
- les monomères vinyliques de formule (I) suivante : 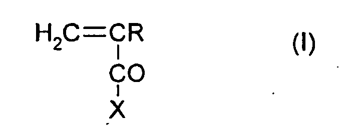
dans laquelle :
- R est choisi parmi H, -CH₃, -C₂H₅ ou -C₃H₇, et
- X est choisi parmi :
- les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor); un groupement sulfonique (-SO₃⁻), sulfate (-SO₄⁻), phosphate (-PO₄H₂) ; hydroxy (-OH) ; amine primaire (-NH₂); amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R'+ R₁ + R₂ + R₃ ne dépasse pas 7 ; et
- les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂) ; amine primaire (-NH₂) ; amine secondaire (NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
- l'anhydride maléfique
- l'acide itaconique ;
- l'alcool vinylique de formule CH₂=CHOH ;
- l'acétate de vinyle de formule CH₂=CH-OCOCH₃ ;
- les N-vinyllactames tels que la N-vinylpyrrolidone, la N-vinylcaprolactame et la N-butyrolactame ;
- les vinyléthers de formule CH₂=CHOR₆ dans laquelle R₆ est un radical hydrocarboné, linéaire ou ramifié, saturé ou insaturé, ayant de 1 à 6 atomes de carbone ;
- les dérivés hydrosolubles du styrène, notamment le styrène sulfonate ;
- le chlorure de diméthyldiallyl ammonium ; et
- la vinylacétamide.

27. Emulsion selon - l'une quelconque des revendications 23 à 25, dans laquelle les unités hydrosolubles sont constituées en tout ou en partie d'un ou plusieurs des composants suivants :
- les polyuréthannes hydrosolubles,
- la gomme de xanthane,
- les alginates et leurs dérivés tels que l'alginate de propylèneglycol,
- les dérivés de cellulose et notamment la carboxyméthylcellulose, l'hydroxypropylcellulose, l'hydroxyéthylcellulose et l'hydroxyéthylcellulose quaternisée,
- les galactomannanes et leurs dérivés tels que la gomme Konjac, la gomme de guar, l'hydroxypropylguar, l'hydroxypropylguar modifié par des groupements méthylcarboxylate de sodium, et le chlorure de guar hydroxypropyl triméthyl ammonium, et
- la polyéthylène-imine.

28. Emulsion selon l'une quelconque des revendications 23 à 27, dans laquelle les unités hydrosolubles ont une masse molaire allant de 1 000 g/mole à 5 000 000 g/mole lorsqu'elles constituent le squelette hydrosoluble d'un polymère greffé, ou une masse molaire allant de 500 g/mole à 100 000 g/mole lorsqu'elles constituent un bloc d'un polymère multiblocs ou lorsqu'elles constituent les greffons d'un polymère greffé.

29. Emulsion selon l'une quelconque des revendications 23 à 28, dans laquelle les unités à LCST sont constituées d'un ou plusieurs des polymères suivants :
- les polyéthers tels que le polyoxyde d'éthylène (POE), le polyoxyde de propylène (POP), et les copolymères statistiques d'oxyde d'éthylène (OE) et d'oxyde de propylène (OP),
- les polyvinylméthyléthers,
- les dérivés polymériques N-substitués de l'acrylamide tels que le poly N-isopropyl acrylamide, le poly N-éthyl acrylamide et les copolymères de N-isopropyl acrylamide ou de N-éthyl acrylamide et d'un monomère vinylique répondant à la formule (I) donnée dans la revendication 9, ou d'un monomère choisi parmi l'anhydride maléique, l'acide itaconique, la vinylpyrrolidone, le styrène et ses dérivés, le chlorure de diméthyldiallyl ammonium, la vinylacétamide, les vinyléthers et les dérivés de l'acétate de vinyle ; et
- le polyvinylcaprolactame et les copolymères de vinyl caprolactame et d'un monomère vinylique répondant à la formule (I) donnée dans la revendication 9, ou d'un monomère choisi parmi l'anhydride maléique, l'acide itaconique, la vinylpyrrolidone, le styrène et ses dérivés, le chlorure de diméthyldiallyl ammonium, la vinylacétamide, les vinyléthers et les dérivés de l'acétate de vinyle.

30. Emulsion selon l'une quelconque - des revendications 23 à 28, dans laquelle les unités à LCST sont constituées de poly oxyde de propylène de formule (POP)ₙ, n étant un nombre entier de 10 à 50, ou de copolymères statistiques d'oxyde d'éthylène (OE) et d'oxyde de propylène (OP), représentés par la formule :
(OE)ₘ (OP)ₙ
dans laquelle m est un nombre entier allant de 1 à 40, de préférence de 2 à 20, et n est un nombre entier allant de 10 à 60, de préférence de 20 à 50.

31. Emulsion selon la revendication 30, dans laquelle la masse molaire des unités à LCST est de 500 à 5 300 g/mole, de préférence de 1 500 à 4 000 g/mole.

32. Emulsion selon la revendication 29, dans laquelle les unités à LCST sont constituées par le poly-N-isopropylacrylamide ou le poly-N-éthylacrylamide, ou un copolymère de N-isopropylamide ou de N-éthylacrylamide et d'un monomère répondant à la formule (I) donnée dans la revendication 9, ou d'un monomère choisi parmi l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

33. Emulsion selon la revendication 32, dans laquelle la masse molaire des unités à LCST est de 1 000 g/mole à 500 000 g/mole, de préférence de 2 000 à 50 000 g/mole.

34. Emulsion selon l'une quelconque des revendications 23 à 28, dans laquelle les unités à LCST sont constituées par un polyvinylcaprolactame ou un copolymère de vinylcaprolactame et d'un monomère vinylique répondant à la formule (I) donnée dans la revendication 9, ou d'un monomère choisi parmi l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

35. Emulsion selon la revendication 34, dans laquelle la masse molaire des unités à LCST est de 1 000 à 500 000 g/mol, de préférence de 2 000 à 50 000 g/mole.

36. Emulsion selon l'une quelconque des revendications 23 à 35, dans laquelle la proportion massique des unités à LCST du polymère est de 5 à 70%, de préférence de 20 à 65% et mieux encore de 30 à 60%, par rapport au polymère.

37. Emulsion selon l'une quelconque des revendications 23 à 36, dans laquelle la température de démixtion des unités à LCST est de 5 à 40°C pour une concentration massique des unités à LCST dans l'eau de 1%.

38. Emulsion selon l'une quelconque des revendication 23 à 37, dans laquelle la concentration massique en polymère de la phase aqueuse est inférieure ou égale à 5%, de préférence de 0,01 à 5%.

39. Emulsion selon l'une quelconque des revendications 23 à 38, dans laquelle la phase aqueuse comprend en outre un tensioactif émulsionnant à une concentration ne dépassant pas 1%.

40. Emulsion selon l'une quelconque des revendications 23 à 39, comprenant de plus un agent gélifiant.

41. Utilisation cosmétique de la composition moussante selon l'une quelconque des revendications 7 à 22, pour le nettoyage et/ou le démaquillage de la peau, y compris le cuir chevelu, les ongles, les cheveux, les cils, les sourcils, les yeux, les muqueuses et les semi-muqueuses, et toute autre zone cutanée du corps et du visage.

42. Utilisation cosmétique d'une émulsion cosmétique selon l'une quelconque des revendications 23 à 40, pour le traitement, le soin, la protection et/ou le maquillage de la peau du visage et/ou du corps, des muqueuses (lèvres), du cuir chevelu et/ou des fibres kératiniques.

43. Procédé cosmétique de nettoyage et/ou de démaquillage de la peau, du cuir chevelu et/ou des cheveux, **caractérisé par le fait qu'**on applique la composition de l'invention, sur la peau, sur le cuir chevelu et/ou sur les cheveux, en présence d'eau, et qu'on élimine la mousse formée et les résidus de salissure par rinçage à l'eau.

## Claims

1. Use of a polymer comprising water-soluble units and units with an LCST, the units with an LCST having in water a demixing temperature of from 5 to 40°C at a concentration of 1% by mass in water, to lower the surface tension or the interface tension of water.

2. Use according to Claim 1, in which the lowering of the surface tension or of the interface tension of water is of at least 15 mN/m for a concentration of polymer in water of 0.1% by mass in the temperature range from 5 to 80°C.

3. Use according to Claim 1, in which the lowering of the surface tension or of the interface tension of water is of at least 20 mN/m for a concentration of polymer in water of 0.1% by mass when the temperature is higher than the demixing temperature of the units with an LCST at this concentration.

4. Use of a polymer comprising water-soluble units and units with an LCST, the units with an LCST having in water a demixing temperature of from 5 to 40°C at a concentration of 1% by mass in water, to manufacture a foam.

5. Use of a polymer comprising water-soluble units and units with an LCST, the units with an LCST having in water a demixing temperature of from 5 to 40°C at a concentration of 1% by mass in water, to manufacture a foam, also comprising a foaming surfactant at a concentration of less than or equal to 5% by mass.

6. Use of a polymer comprising water-soluble units and units with an LCST, the units with an LCST having in water a demixing temperature of from 5 to 40°C at a concentration of 1% by mass in water, to manufacture an emulsion free of additional emulsifying surfactant or containing an additional emulsifying surfactant at a concentration of less than or equal to 1% by mass.

7. Foaming composition comprising an aqueous phase containing a polymer comprising water-soluble units and units with an LCST, the units with an LCST having in water a demixing temperature of from 5 to 40°C at a concentration of 1% by mass in water.

8. Foaming composition according to Claim 7, in which the polymer is in the form of a block polymer comprising water-soluble units alternating with units with an LCST, or in the form of a grafted polymer whose backbone is formed from water-soluble units and which bears grafts consisting of units with an LCST, this structure possibly being partially crosslinked, or alternatively in the form of a grafted polymer whose backbone is formed from units with an LCST and which bears grafts consisting of water-soluble units, this structure possibly being partially crosslinked.

9. Foaming composition according to either of Claims 7 and 8, in which the water-soluble units are obtained by free-radical polymerization of at least one monomer chosen from:
- (meth) acrylic acid;
- vinyl monomers of formula (I) below: 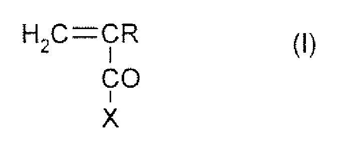
in which:
- R is chosen from H, -CH₃, -C₂H₅ or -C₃H₇, and
- X is chosen from:
- alkyl oxides of -OR' type in which R' is a linear or branched, saturated or unsaturated hydrocarbon radical containing from 1 to 6 carbon atoms, optionally substituted with at least one halogen atom (iodine, bromine, chlorine or fluorine); sulphonic (-SO₃⁻), sulphate (-SO₄⁻) , phosphate (-PO₄H₂); hydroxyl (-OH); primary amine (-NH₂) ; secondary amine (-NHR₁), tertiary amine (-NR₁R₂) or quaternary amine (-N⁺R₁R₂R₃) group with R₁, R₂ and R₃ being, independently of each other, a linear or branched, saturated or unsaturated hydrocarbon radical containing 1 to 6 carbon atoms, with the proviso that the sum of the carbon atoms of R' + R₁ + R₂ + R₃ does not exceed 7; and
- -NH₂, -NHR₄ and -NR₄R₅ groups in which R₄ and R₅ are, independently of each other, linear or branched, saturated or unsaturated hydrocarbon radicals containing 1 to 6 carbon atoms, with the proviso that the total number of carbon atoms in R₄ + R₅ does not exceed 7, the said R₄ and R₅ optionally being substituted with a halogen atom (iodine, bromine, chlorine or fluorine); a hydroxyl (-OH); sulphonic (-SO₃⁻), sulphate (-SO₄⁻) ; phosphate (-PO₄H₂); primary amine (-NH₂); secondary amine (-NHR₁), tertiary amine (-NR₁R₂) and/or quaternary amine (-N⁺R₁R₂R₃) group with R₁, R₂ and R₃ being, independently of each other, a linear or branched, saturated or unsaturated hydrocarbon radical containing 1 to 6 carbon atoms, with the proviso that the sum of the carbon atoms of R₄ + R₅ + R₁ + R₂ + R₃ does not exceed 7;
- maleic anhydride;
- itaconic acid;
- vinyl alcohol of formula CH₂=CHOH;
- vinyl acetate of formula CH₂=CH-OCOCH₃;
- N-vinyllactams such as N-vinylpyrrolidone, N-vinylcaprolactam and N-butyrolactam;
- vinyl ethers of formula CH₂=CHOR₆ in which R₆ is a linear or branched, saturated or unsaturated hydrocarbon radical containing from 1 to 6 carbon atoms;
- water-soluble styrene derivatives, especially styrene sulphonate;
- dimethyldiallylammonium chloride; and
- vinylacetamide.

10. Foaming composition according to either of Claims 7 and 8, in which the water-soluble units consist totally or partially of one or more of the following components:
- water-soluble polyurethanes,
- xanthan gum,
- alginates and derivatives thereof such as propylene glycol alginate,
- cellulose derivatives and especially carboxymethylcellulose, hydroxypropylcellulose, hydroxyethylcellulose and quaternized hydroxyethylcellulose,
- galactomannans and derivatives thereof such as konjac gum, guar gum, hydroxypropylguar, hydroxypropylguar modified with sodium methylcarboxylate groups, and hydroxypropyltrimethylammonium guar chloride, and
- polyethyleneimine.

11. Foaming composition according to any one of Claims 7 to 10, in which the water-soluble units have a molar mass ranging from 1000 g/mol to 5 000 000 g/mol when they constitute the water-soluble backbone of a grafted polymer, or a molar mass ranging from 500 g/mol to 100 000 g/mol when they constitute one block of a multiblock polymer or when they constitute the grafts of a grafted polymer.

12. Foaming composition according to any one of Claims 7 to 11, in which the units with an LCST consist of one or more of the following polymers:
- polyethers such as polyethylene oxide (PEO), polypropylene oxide (PPO) and random copolymers of ethylene oxide (EO) and of propylene oxide (PO),
- polyvinyl methyl ethers,
- polymeric N-substituted acrylamide derivatives such as poly-N-isopropylacrylamide, poly-N-ethylacrylamide and copolymers of N-isopropylacrylamide or of N-ethylacrylamide and of a vinyl monomer corresponding to formula (I) given in Claim 9, or of a monomer chosen from maleic anhydride, itaconic acid, vinylpyrrolidone, styrene and its derivatives, dimethyldiallylammonium chloride, vinylacetamide, vinyl ethers and vinyl acetate derivatives; and
- polyvinylcaprolactam and copolymers of vinylcaprolactam and of a vinyl monomer corresponding to formula (I) given in Claim 9, or of a monomer chosen from maleic anhydride, itaconic acid, vinylpyrrolidone, styrene and its derivatives, dimethyldiallylammonium chloride, vinylacetamide, vinyl ethers and vinyl acetate derivatives.

13. Foaming composition according to any one of Claims 7 to 11, in which the units with an LCST consist of polypropylene oxides of formula (PPO)ₙ with n being an integer from 10 to 50, or random copolymers of ethylene oxide (EO) and of propylene oxide (PO), represented by the formula:
(EO)ₘ(PO)n
in which m is an integer ranging from 1 to 40 and preferably from 2 to 20, and n is an integer ranging from 10 to 60 and preferably from 20 to 50.

14. Foaming composition according to Claim 13, in which the molar mass of the units with an LCST is from 500 to 5300 g/mol and preferably from 1500 to 4000 g/mol.

15. Foaming composition according to Claim 12, in which the units with an LCST consist of poly-N-isopropylamide or poly-N-ethylacrylamide or a copolymer of N-isopropylacrylamide or of N-ethylacrylamide and of a monomer corresponding to formula (I) given in Claim 9, or of a monomer chosen from maleic anhydride, itaconic acid, vinylpyrrolidone, styrene and its derivatives, dimethyldiallylammonium chloride, vinylacetamide, vinyl alcohol, vinyl acetate, vinyl ethers and vinyl acetate derivatives.

16. Foaming composition according to Claim 15, in which the molar mass of the units with an LCST is from 1000 g/mol to 500 000 g/mol and preferably from 2000 to 50 000 g/mol.

17. Foaming composition according to any one of Claims 7 to 11, in which the units with an LCST consist of a polyvinylcaprolactam or a copolymer of vinylcaprolactam and of a vinyl monomer corresponding to formula (I) given in Claim 9, or of a monomer chosen from maleic anhydride, itaconic acid, vinylpyrrolidone, styrene and its derivatives, dimethyldiallylammonium chloride, vinylacetamide, vinyl alcohol, vinyl acetate, vinyl ethers and vinyl acetate derivatives.

18. Foaming composition according to Claim 17, in which the molar mass of the units with an LCST is from 1000 to 500 000 g/mol and preferably from 2000 to 50 000 g/mol.

19. Foaming composition according to any one of Claims 7 to 18, in which the proportion by mass of the units with an LCST of the polymer is from 5 to 70%, preferably from 20 to 65% and better still from 30 to 60% relative to the polymer.

20. Foaming composition according to any one of Claims 7 to 19, in which the demixing temperature of the units with an LCST is from 5 to 40°C for a concentration of the units with an LCST in water of 1% by mass.

21. Foaming composition according to any one of Claims 7 to 20, in which the concentration by mass of polymer in the aqueous phase is less than or equal to 5% and preferably from 0.01% to 5%.

22. Foaming composition according to any one Claims 7 to 21, in which the aqueous phase also comprises a foaming surfactant in a concentration not exceeding 5% by mass.

23. Oil-in-water emulsion comprising an aqueous phase and an oily phase dispersed in the aqueous phase, in which the aqueous phase comprises a polymer comprising water-soluble units and units with an LCST, the units with an LCST having in water a demixing temperature of from 5 to 40°C at a concentration of 1% by mass in water.

24. Water-in-oil-in-water emulsion comprising a water-in-oil emulsion dispersed in an outer aqueous phase, in which the outer aqueous phase comprises a polymer comprising water-soluble units and units with an LCST, the units with an LCST having in water a demixing temperature of from 5 to 40°C at a concentration of 1% by mass in water.

25. Emulsion according to Claim 23 or 24, in which the polymer is in the form of a block polymer comprising water-soluble units alternating with units with an LCST, or in the form of a grafted polymer whose backbone is formed from water-soluble units and which bears grafts consisting of units with an LCST, this structure possibly being partially crosslinked, or alternatively in the form of a grafted polymer whose backbone is formed from units with an LCST and which bears grafts consisting of water-soluble units, this structure possibly being partially crosslinked.

26. Emulsion according to any one of Claims 23 to 25, in which the water-soluble units are obtained by free-radical polymerization of at least one monomer chosen from:
- (meth) acrylic acid;
- vinyl monomers of formula (I) below: 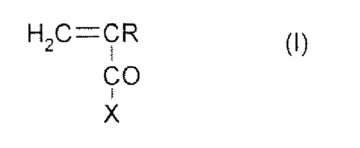
in which:
- R is chosen from H, -CH₃, -C₂H₅ or -C₃H₇, and
- X is chosen from:
- alkyl oxides of -OR' type in which R' is a linear or branched, saturated or unsaturated hydrocarbon radical containing from 1 to 6 carbon atoms, optionally substituted with at least one halogen atom (iodine, bromine, chlorine or fluorine); sulphonic (-SO₃⁻) sulphate (-SO₄⁻) , phosphate (-PO₄H₂) ; hydroxyl (-OH); primary amine (-NH₂); secondary amine (-NHR₁) , tertiary amine (-NR₁R₂) or quaternary amine (-N⁺R₁R₂R₃) group with R₁, R₂ and R₃ being, independently of each other, a linear or branched, saturated or unsaturated hydrocarbon radical containing 1 to 6 carbon atoms, with the proviso that the sum of the carbon atoms of R' + R₁ + R₂ + R₃ does not exceed 7; and
- -NH₂, -NHR₄ and -NR₄R₅ groups in which R₄ and R₅ are, independently of each other, linear or branched, saturated or unsaturated hydrocarbon radicals containing 1 to 6 carbon atoms, with the proviso that the total number of carbon atoms in R₄ + R₅ does not exceed 7, the said R₄ and R₅ optionally being substituted with a halogen atom (iodine, bromine, chlorine or fluorine); a hydroxyl (-OH); sulphonic (-SO₃⁻) sulphate (-SO₄⁻) ; phosphate (-PO₄H₂) ; primary amine (-NH₂) ; secondary amine (-NHR₁) , tertiary amine (-NR₁R₂) and/or quaternary amine (-N⁺R₁R₂R₃) group with R₁, R₂ and R₃ being, independently of each other, a linear or branched, saturated or unsaturated hydrocarbon radical containing 1 to 6 carbon atoms, with the proviso that the sum of the carbon atoms of R₄ + R₅ + R₁ + R₂ + R₃ does not exceed 7;
- maleic anhydride;
- itaconic acid;
- vinyl alcohol of formula CH₂=CHOH;
- vinyl acetate of formula CH₂=CH-OCOCH₃;
- N-vinyllactams such as N-vinylpyrrolidone, N-vinylcaprolactam and N-butyrolactam;
- vinyl ethers of formula CH₂=CHOR₆ in which R₆ is a linear or branched, saturated or unsaturated hydrocarbon radical containing from 1 to 6 carbon atoms;
- water-soluble styrene derivatives, especially styrene sulphonate;
- dimethyldiallylammonium chloride; and
- vinylacetamide.

27. Emulsion according to any one of Claims 23 to 25, in which the water-soluble units consist totally or partially of one or more of the following components:
- water-soluble polyurethanes,
- xanthan gum,
- alginates and derivatives thereof such as propylene glycol alginate,
- cellulose derivatives and especially carboxymethylcellulose, hydroxypropylcellulose, hydroxyethylcellulose and quaternized hydroxyethylcellulose,
- galactomannans and derivatives thereof such as konjac gum, guar gum, hydroxypropylguar, hydroxypropylguar modified with sodium methylcarboxylate groups, and hydroxypropyltrimethylammonium guar chloride, and
- polyethyleneimine.

28. Emulsion according to any one of Claims 23 to 27, in which the water-soluble units have a molar mass ranging from 1000 g/mol to 5 000 000 g/mol when they constitute the water-soluble backbone of a grafted polymer, or a molar mass ranging from 500 g/mol to 100 000 g/mol when they constitute one block of a multiblock polymer or when they constitute the grafts of a grafted polymer.

29. Emulsion according to any one of Claims 23 to 28, in which the units with an LCST consist of one or more of the following polymers:
- polyethers such as polyethylene oxide (PEO), polypropylene oxide (PPO) and random copolymers of ethylene oxide (EO) and of propylene oxide (PO),
- polyvinyl methyl ethers,
- polymeric N-substituted acrylamide derivatives such as poly-N-isopropylacrylamide, poly-N-ethylacrylamide and copolymers of N-isopropylacrylamide or of N-ethylacrylamide and of a vinyl monomer corresponding to formula (I) given in Claim 9, or of a monomer chosen from maleic anhydride, itaconic acid, vinylpyrrolidone, styrene and its derivatives, dimethyldiallylammonium chloride, vinylacetamide, vinyl ethers and vinyl acetate derivatives; and
- polyvinylcaprolactam and copolymers of vinylcaprolactam and of a vinyl monomer corresponding to formula (I) given in Claim 9, or of a monomer chosen from maleic anhydride, itaconic acid, vinylpyrrolidone, styrene and its derivatives, dimethyldiallylammonium chloride, vinylacetamide, vinyl ethers and vinyl acetate derivatives.

30. Emulsion according to any one of Claims 23 to 28, in which the units with an LCST consist of polypropylene oxide of formula (PPO)ₙ with n being an integer from 10 to 50, or random copolymers of ethylene oxide (EO) and of propylene oxide (PO), represented by the formula:
(EO)ₘ(PO)ₙ
in which m is an integer ranging from 1 to 40 and preferably from 2 to 20, and n is an integer ranging from 10 to 60 and preferably from 20 to 50.

31. Emulsion according to Claim 30, in which the molar mass of the units with an LCST is from 500 to 5300 g/mol and preferably from 1500 to 4000 g/mol.

32. Emulsion according to Claim 29, in which the units with an LCST consist of poly-N-isopropylacrylamide or poly-N-ethylacrylamide or a copolymer of N-isopropylamide or of N-ethylacrylamide and of a monomer corresponding to formula (I) given in Claim 9, or of a monomer chosen from maleic anhydride, itaconic acid, vinylpyrrolidone, styrene and its derivatives, dimethyldiallylammonium chloride, vinylacetamide, vinyl alcohol, vinyl acetate, vinyl ethers and vinyl acetate derivatives.

33. Emulsion according to Claim 32, in which the molar mass of the units with an LCST is from 1000 g/mol to 500 000 g/mol and preferably from 2000 to 50 000 g/mol.

34. Emulsion according to any one of Claims 23 to 28, in which the units with an LCST consist of a polyvinylcaprolactam or a copolymer of vinylcaprolactam and of a vinyl monomer corresponding to formula (I) given in Claim 9, or of a monomer chosen from maleic anhydride, itaconic acid, vinylpyrrolidone, styrene and its derivatives, dimethyldiallylammonium chloride, vinylacetamide, vinyl alcohol, vinyl acetate, vinyl ethers and vinyl acetate derivatives.

35. Emulsion according to Claim 34, in which the molar mass of the units with an LCST is from 1000 to 500 000 g/mol and preferably from 2000 to 50 000 g/mol.

36. Emulsion according to any one of Claims 23 to 35, in which the proportion by mass of the units with an LCST of the polymer is from 5 to 70%, preferably from 20 to 65% and better still from 30 to 60% relative to the polymer.

37. Emulsion according to any one of Claims 23 to 36, in which the demixing temperature of the units with an LCST is from 5 to 40°C for a concentration of the units with an LCST in water of 1% by mass.

38. Emulsion according to any one of Claims 23 to 37, in which the concentration by mass of polymer in the aqueous phase is less than or equal to 5% and preferably from 0.01% to 5%.

39. Emulsion according to any one of Claims 23 to 38, in which the aqueous phase also comprises an emulsifying surfactant at a concentration not exceeding 1%.

40. Emulsion according to any one of Claims 23 to 39, also comprising a gelling agent.

41. Cosmetic use of the foaming composition according to any one of Claims 7 to 22, for cleansing and/or removing make-up from the skin, including the scalp, the nails, the hair, the eyelashes, the eyebrows, the eyes, mucous membranes and semi-mucous membranes, and any other area of body or facial skin.

42. Cosmetic use of a cosmetic emulsion according to any one of Claims 23 to 40, for treating, caring for, protecting and/or making up facial skin and/or body skin, mucous membranes (lips), the scalp and/or keratin fibres.

43. Cosmetic process for cleansing and/or removing make-up from the skin, the scalp and/or the hair, **characterized in that** the composition of the invention is applied to the skin, to the scalp and/or to the hair, in the presence of water, and the foam formed and the soiling residues are removed by rinsing with water.

## Patentansprüche

1. Verwendung eines Polymers, das wasserlösliche Einheiten und Einheiten mit einer unteren kritischen Lösungstemperatur (lower critical solution temperature, LCST) aufweist, wobei die LCST-Einheiten für eine Massekonzentration in Wasser von 1 % eine Entmischungstemperatur in Wasser von 5 bis 40 °C aufweisen, um die Oberflächenspannung oder Grenzflächenspannung des Wassers zu vermindern.

2. Verwendung nach Anspruch 1, wobei die Absenkung der Oberflächenspannung oder Grenzflächenspannung des Wassers für eine Massekonzentration des Polymers in Wasser von 0,1 % im Temperaturbereich von 5 bis 80 °C mindestens 15 mN/m beträgt.

3. Verwendung nach Anspruch 1, wobei die Absenkung der Oberflächenspannung oder Grenzflächenspannung des Wassers für eine Massekonzentration des Polymers in Wasser von 0,1 % mindestens 20 mN/m beträgt, wenn die Temperatur über der Entmischungstemperatur der LCST-Einheiten bei dieser Konzentration liegt.

4. Verwendung eines Polymers, das wasserlösliche Einheiten und LCST-Einheiten aufweist, wobei die LCST-Einheiten für eine Massekonzentration in Wasser von 1 % eine Entmischungstemperatur in Wasser von 5 bis 40 °C aufweisen, um einen Schaum zu bilden.

5. Verwendung eines Polymers, das wasserlösliche Einheiten und LCST-Einheiten aufweist, wobei die LCST-Einheiten für eine Massekonzentration in Wasser von 1 % eine Entmischungstemperatur in Wasser von 5 bis 40 °C aufweisen, um einen Schaum zu bilden, der ferner einen schaumbildenden grenzflächenaktiven Stoff in einer Massekonzentration von 5 % oder darunter auf weist.

6. Verwendung eines Polymers, das wasserlösliche Einheiten und LCST-Einheiten aufweist, wobei die LCST-Einheiten für eine Massekonzentration in Wasser von 1 % eine Entmischungstemperatur in Wasser von 5 bis 40 °C aufweisen, um eine Emulsion zu bilden, die keinen ergänzenden emulgierenden grenzflächenaktiven Stoff oder einen ergänzenden emulgierenden grenzflächenaktiven Stoff in einer Massekonzentration von 1 % oder darunter aufweist.

7. Schaumbildende Zusammensetzung, die eine wässrige Phase aufweist, die ein Polymer enthält, das wasserlösliche Einheiten und LCST-Einheiten aufweist, wobei die LCST-Einheiten für eine Massekonzentration in Wasser von 1 % eine Entmischungstemperatur in Wasser von 5 bis 40 °C aufweisen.

8. Schaumbildende Zusammensetzung nach Anspruch 7, wobei das Polymer in Form eines Sequenzpolymers (oder Blockpolymers), das wasserlösliche Einheiten aufweist, die mit LCST-Einheiten alternieren, oder in Form eines Pfropfpolymers, dessen Grundgerüst aus wasserlöslichen Einheiten gebildet wird und Pfropfzweige trägt, die aus LCST-Einheiten bestehen, wobei diese Struktur partiell vernetzt sein kann, oder in Form eines Pfropfpolymers vorliegt, dessen Grundgerüst aus LCST-Einheiten gebildet wird und Pfropfzweige trägt, die aus wasserlöslichen Einheiten bestehen, wobei diese Struktur partiell vernetzt sein kann.

9. Schaumbildende Zusammensetzung nach einem der Ansprüche 7 und 8, in der die wasserlöslichen Einheiten durch radikalische Polymerisation mindestens eines Monomers gebildet sind, das unter den folgenden Monomeren ausgewählt ist:
- (Meth)acrylsäure;
- Vinylmonomeren der folgenden Formel (I): 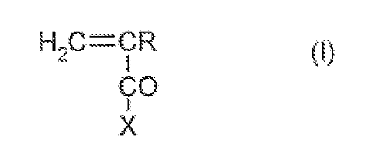 in der Formel:
- R ist unter H, -CH₃, -CH₂H₅ oder -C₃H₇ ausgewählt und
- X ist ausgewählt unter:
·alkoxygruppen vom Typ -OR', wobei R' eine geradkettige oder verzweigte, gesättigte oder ungesättigte Kohlenwasserstoffgruppe mit 1 bis 6 Kohlenstoffatomen bedeutet, die gegebenenfalls substituiert ist mit mindestens einem Halogenatom (Iod, Brom, Chlor, Fluor); einer Sulfonsäuregruppe (-SO₃⁻), einer Sulfatgruppe (-SO₄-), einer Phosphatgruppe (-PO₄H₂); einer Hydroxygruppe (-OH); einer primären Aminogruppe (-NH₂); einer sekundären Aminogruppe (-NHR₁), einer tertiären Aminogruppe (-NR₁R₂) oder einer quartären Aminogruppe (-N⁺R₁R₂R₅), wobei R₁, R₂ und R₃ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Anzahl der Kohlenstoffatome von R' + R₁ + R₂ + R₃ 7 nicht übersteigt; und
·den Gruppen -NH₂, -NHR₄ und -NR₄R₅, wobei R₄ und R₅ unabhängig voneinander geradkettige oder verzweigte, gesättigte oder ungesättigte Kohlenwasserstoffgruppen mit 1 bis 6 Kohlenstoffatomen bedeuten, mit der Maßgabe, dass die Gesamtzahl der Kohlenstoffatome von R₄ + R₅ 7 nicht übersteigt, wobei R₄ und R₅ gegebenenfalls substituiert sind mit einem Halogenatom (Iod, Brom, Chlor, Fluor); einer Hydroxygruppe (-OH); einer Sulfonsäuregruppe (-SO₃⁻); einer Sulfatgruppe (-SO₄⁻ ); einer Phosphatgruppe (-PO₄H₂); einer primären Aminogruppe (-NH₂); einer sekundären Aminogruppe (-NHR₁), einer tertiären Aminogruppe (-NR₁R₂) und/oder einer quartären Aminogruppe (-N⁺R₁R₂R₃) , wobei R₁, R₂ und R₃ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₄ + R₅ + R₁ + R₂ + R₃ 7 nicht übersteigt;
- Maleinsäureanhydrid;
- Itaconsäure;
- Vinylalkohol der Formel CH₂=CHOH;
- Vinylacetat der Formel CH₂=CH-OCOCH₃;
- N-Vinyllactamen, wie N-Vinylpyrrolidon, N-Vinylcaprolactam und N-Butyrolactam;
- Vinylethern der Formel CH₂=CHOR₆, wobei R₆ eine geradkettige oder verzweige, gesättigte oder ungesättigte Kohlenwasserstoffgruppe mit 1 bis 6 Kohlenstoffatomen ist;
- wasserlöslichen Styrolderivaten, insbesondere Styrolsulfonat;
- Dimethyldiallylammoniumchlorid;
und
- Vinylacetamid.

10. Schaumbildende Zusammensetzung nach einem der Ansprüche 7 und 8, wobei die wasserlöslichen Einheiten ganz oder teilweise aus einer oder mehreren der folgenden Komponenten aufgebaut sind:
- wasserlöslichen Polyurethanen;
- Xanthangummi;
- Alginaten und deren Derivaten, wie Propylenglycolalginat;
- Cellulosederivaten und insbesondere Carboxymethylcellulose, Hydroxypropylcellulose, Hydroxyethylcellulose und quaternisierter Hydroxyethylcellulose;
- Galactomannanen und deren Derivaten, wie Konjac-Gummi, Guargummi, Hydroxypropylguar, mit Natriummethylcarboxylatgruppen modifiziertes Hydroxypropylguargummi und Guarhydroxypropyltrimethylammoniumchlorid; und
- Polyethylenimin.

11. Schaumbildende Zusammensetzung nach einem der Ansprüche 7 bis 10, wobei die wasserlöslichen Einheiten eine Molmasse von 1 000 bis 5 000 000 g/mol aufweisen, wenn sie das wasserlösliche Grundgerüst eines Pfropfpolymers bilden, oder eine Molmasse von 500 bis 100 000 g/mol, wenn sie einen Block eines Multiblockpolymers bilden oder wenn sie die Pfropfzweige eines Pfropf polymers bilden.

12. Schaumbildende Zusammensetzung nach einem der Ansprüche 7 bis 11, wobei die LCST-Einheiten aus einem oder mehreren der folgenden Polymere bestehen:
- Polyethern, wie Polyethylenoxid (PEO), Polypropylenoxid (PPO) und statistischen Copolymeren von Ethylenoxid (EO) und Propylenoxid (PO);
- Polyvinylmethylethern;
- N-substituierten Polymerderivaten von Acrylamid, wie Poly-N-Isopropylacrylamid, Poly-N-Ethylacrylamid und Copolymeren von N-Isopropylacrylamid oder N-Ethylacrylamid und einem Vinylmonomer der Formel (I), die in Anspruch 9 angegeben ist, oder einem Monomer, das unter Maleinsäureanhydrid, Itaconsäure, Vinylpyrrolidon, Styrol und seinen Derivaten, Dimethyldiallylammoniumchlorid, Vinylacetamid, Vinylethern und Derivaten von Vinylacetat ausgewählt ist; und
- Polyvinylcaprolactam und Copolymeren von Vinylcaprolactam und einem Vinylmonomer der Formel (I), die in Anspruch 9 angegeben ist, oder einem Monomer, das unter Maleinsäureanhydrid, Itaconsäure, Vinylpyrrolidon, Styrol und seinen Derivaten, Dimethyldiallylammoniumchlorid, Vinylacetamid, Vinylethern und Derivaten von Vinylacetat ausgewählt ist.

13. Schaumbildende Zusammensetzung nach einem der Ansprüche 7 bis 11, wobei die LCST-Einheiten aus Polypropylenoxid der Formel (PPO)ₙ bestehen, wobei n eine ganze Zahl von 10 bis 50 bedeutet, oder statistischen Copolymeren von Ethylenoxid (EO) und Propylenoxid (PO), die durch die folgende Formel dargestellt werden:
(EO)ₘ (PO)ₙ,
wobei m eine ganze Zahl von 1 bis 40 und vorzugsweise 2 bis 20 bedeutet und n eine ganze Zahl von 10 bis 60 und vorzugsweise 20 bis 50 ist.

14. Schaumbildende Zusammensetzung nach Anspruch 13, wobei die Molmasse der LCST-Einheiten 500 bis 5 300 g/mol und vorzugsweise 1 500 bis 4 000 g/mol beträgt.

15. Schaumbildende Zusammensetzung nach Anspruch 12, wobei die LCST-Einheiten von Poly-N-Isopropylacrylamid oder Poly-N-Ethylacrylamid und den Copolymeren von N-Isopropylacrylamid oder N-Ethylacrylamid und einem Monomer, das der in Anspruch 9 angegebenen Formel (I) entspricht, oder einem Monomer, das unter Maleinsäureanhydrid, Itaconsäure, Vinylpyrrolidon, Styrol und seinen Derivaten, Dimethyldiallylammoniumchlorid, Vinylacetamid, Vinylalkohol, Vinylacetat, Vinylethern und Vinylacetatderivaten ausgewählt ist.

16. Schaumbildende Zusammensetzung nach Anspruch 15, wobei die Molmasse der LCST-Einheiten 1 000 bis 500 000 g/mol und vorzugsweise 2 000 bis 50 000 g/mol beträgt.

17. Schaumbildende Zusammensetzung nach einem der Ansprüche 7 bis 11, wobei die LCST-Einheiten gebildet sind aus einem Polyvinylcaprolactam oder einem Copolymer von Vinylcaprolactam und einem Vinylmonomer der in Anspruch 9 angegebenen Formel (I) oder einem Monomer gebildet sind, das unter Maleinsäureanhydrid, Itaconsäure, Vinylpyrrolidon, Styrol und seinen Derivaten, Dimethyldiallylammoniumchlorid, Vinylacetamid, Vinylalkohol, Vinylacetat, Vinylethern und Vinylacetatderivaten ausgewählt ist.

18. Schaumbildende Zusammensetzung nach Anspruch 17, wobei die Molmasse der LCST-Einheiten 1 000 bis 500 000 g/mol und vorzugsweise 2 000 bis 50 000 g/mol beträgt.

19. Schaumbildende Zusammensetzung nach einem der Ansprüche 7 bis 18, wobei der Masseanteil der LCST-Einheiten des Polymers 5 bis 70 %, vorzugsweise 20 bis 65 % und besser 30 bis 60 %, bezogen auf das Polymer, beträgt.

20. Schaumbildende Zusammensetzung nach einem der Ansprüche 7 bis 19, wobei die Entmischungstemperatur der LCST-Einheiten für eine Massekonzentration der LCST-Einheiten in Wasser von 1 % 5 bis 40 °C beträgt.

21. Schaumbildende Zusammensetzung nach einem der Ansprüche 7 bis 20, wobei die Massekonzentration des Polymers der wässrigen Phase 5 % oder darunter und vorzugsweise 0,01 bis 5 % beträgt.

22. Schaumbildende Zusammensetzung nach einem der Ansprüche 7 bis 21, bei der die wässrige Phase ferner einen schaumbildenden grenzflächenaktiven Stoff in einer Massekonzentration enthält, die 5 % nicht übersteigt.

23. Öl-in-Wasser-Emulsion, die ein wässrige Phase und eine in der wässrigen Phase dispergierte Ölphase umfasst, wobei die wässrige Phase ein Polymer enthält, das wasserlösliche Einheiten und LCST-Einheiten aufweist, wobei die LCST-Einheiten für eine Massekonzentration in Wasser von 1 % eine Entmischungstemperatur in Wasser von 5 bis 40 °C aufweisen.

24. Wasser-in-Öl-in-Wasser-Emulsion, die eine in einer äußeren wässrigen Phase dispergierte Wasser-in-Öl-Emulsion umfasst,
wobei die äußere wässrige Phase ein Polymer enthält, das wasserlösliche Einheiten und LCST-Einheiten aufweist, wobei die LCST-Einheiten für eine Massekonzentration in Wasser von 1 % eine Entmischungstemperatur in Wasser von 5 bis 40 °C aufweisen.

25. Emulsion nach Anspruch 23 oder 24, wobei das Polymer in Form eines Sequenzpolymers (oder Blockpolymers), das wasserlösliche Einheiten aufweist, die mit LCST-Einheiten alternieren, oder in Form eines Pfropfpolymers, dessen Grundgerüst aus wasserlöslichen Einheiten gebildet wird und Pfropfzweige trägt, die aus LCST-Einheiten bestehen, wobei diese Struktur partiell vernetzt sein kann, oder in Form eines Pfropfpolymers vorliegt, dessen Grundgerüst aus LCST-Einheiten gebildet wird und Pfropfzweige trägt, die aus wasserlöslichen Einheiten bestehen, wobei diese Struktur partiell vernetzt sein kann.

26. Emulsion nach einem der Ansprüche 23 bis 25, in der die wasserlöslichen Einheiten durch radikalische Polymerisation mindestens eines Monomers gebildet sind, das unter den folgenden Monomeren ausgewählt ist:
- (Meth)acrylsäure;
- Vinylmonomeren der folgenden Formel (I): 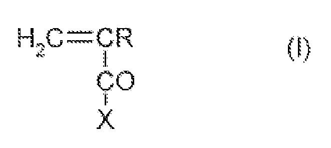 in der Formel:
- R ist unter H, -CH₃, -CH₂H₅ oder -C₃H₇ ausgewählt und
- X ist ausgewählt unter:
-Alkoxygruppen vom Typ -OR', wobei R' eine geradkettige oder verzweigte, gesättigte oder ungesättigte Kohlenwasserstoffgruppe mit 1 bis 6 Kohlenstoffatomen bedeutet, die gegebenenfalls substituiert ist mit mindestens einem Halogenatom (Iod, Brom, Chlor, Fluor); einer Sulfonsäuregruppe (-SO₃⁻), einer Sulfatgruppe (-SO₄⁻), einer Phosphatgruppe (-PO₄H₂); einer Hydroxygruppe (-OH); einer primären Aminogruppe (-NH₂); einer sekundären Aminogruppe (-NHR₁), einer tertiären Aminogruppe (-NR₁R₂) oder einer quartären Aminogruppe (-N⁺R₁R₂R₃), wobei R₁, R₂ und R₃ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Anzahl der Kohlenstoffatome von R' + R₁ + R₂ + R₃ 7 nicht übersteigt; und
den Gruppen -NH₂, -NHR₄ und -NR₄R₅, wobei R₄ und R₅ unabhängig voneinander geradkettige oder verzweigte, gesättigte oder ungesättigte Kohlenwasserstoffgruppen mit 1 bis 6 Kohlenstoffatomen bedeuten, mit der Maßgabe, dass die Gesamtzahl der Kohlenstoffatome von R₄ + R₅ 7 nicht übersteigt, wobei R₄ und R₅ gegebenenfalls substituiert sind mit einem Halogenatom (Iod, Brom, Chlor, Fluor); einer Hydroxygruppe (-OH); einer Sulfonsäuregruppe (-SO₃⁻); einer Sulfatgruppe (-SO₄⁻); einer Phosphatgruppe (-PO₄H₂); einer primären Aminogruppe (-NH₂); einer sekundären Aminogruppe (-NHR₁), einer tertiären Aminogruppe (-NR₁R₂) und/oder einer quartären Aminogruppe (-N⁺R₁R₂R₃), wobei R₁, R₂ und R₃ unabhängig voneinander eine geradkettige oder verzweigte, gesättigte oder ungesättigte Kohlenwasserstoffgruppe mit 1 bis 6 Kohlenstoffatomen bedeuten, mit der Maßgabe, dass die Summe der Kohlenstoffatome von R₄ + R₅ + R₁ + R₂ + R₃ 7 nicht übersteigt;
- Maleinsäureanhydrid;
- Itaconsäure;
- Vinylalkohol der Formel CH₂=CHOH;
- Vinylacetat der Formel CH₂=CH-OCOCH₃;
- N-Vinyllactamen, wie N-Vinylpyrrolidon, N-Vinylcaprolactam und N-Butyrolactam;
- Vinylethern der Formel CH₂=CHOR₆, wobei R₆ eine geradkettige oder verzweige, gesättigte oder ungesättigte Kohlenwasserstoffgruppe mit 1 bis 6 Kohlenstoffatomen ist;
- wasserlöslichen Styrolderivaten, insbesondere Styrolsulfonat;
- Dimethyldiallylammoniumchlorid;
und
- Vinylacetamid.

27. Emulsion nach einem der Ansprüche 23 bis 25, bei der die wasserlöslichen Einheiten ganz oder teilweise aus einer oder mehreren der folgenden Komponenten aufgebaut sind:
- wasserlöslichen Polyurethanen;
- Xanthangummi;
- Alginaten und deren Derivaten, wie Propylenglycolalginat;
- Cellulosederivaten und insbesondere Carboxymethylcellulose, Hydroxypropylcellulose, Hydroxyethylcellulose und quaternisierter Hydroxyethylcellulose;
- Galactomannanen und deren Derivaten, wie Konjac-Gummi, Guargummi, Hydroxypropylguar, mit Natriummethylcarboxylatgruppen modifiziertes Hydroxypropylguargummi und Guarhydroxypropyltrimethylammoniumchlorid; und
- Polyethylenimin.

28. Emulsion nach einem der Ansprüche 23 bis 27, wobei die wasserlöslichen Einheiten eine Molmasse von 1 000 bis 5 000 000 g/mol aufweisen, wenn sie das wasserlösliche Grundgerüst eines Pfropfpolymers bilden, oder eine Molmasse von 500 bis 100 000 g/mol, wenn sie einen Block eines Multiblockpolymers bilden oder wenn sie die Pfropfzweige eines Pfropfpolymers bilden.

29. Emulsion nach einem der Ansprüche 23 bis 28, wobei die LCST-Einheiten aus einem oder mehreren der folgenden Polymere bestehen:
- Polyethern, wie Polyethylenoxid (PEO), Polypropylenoxid (PPO) und statistischen Copolymeren von Ethylenoxid (EO) und Propylenoxid (PO);
- Polyvinylmethylethern;
- N-substituierten Polymerderivaten von Acrylamid, wie Poly-N-Isopropylacrylamid, Poly-N-Ethylacrylamid und Copolymeren von N-Isopropylacrylamid oder N-Ethylacrylamid und einem Vinylmonomer der Formel (I), die in Anspruch 9 angegeben ist, oder einem Monomer, das unter Maleinsäureanhydrid, Itaconsäure, Vinylpyrrolidon, Styrol und seinen Derivaten, Dimethyldiallylammoniumchlorid, Vinylacetamid, Vinylethern und Derivaten von Vinylacetat ausgewählt ist; und
- Polyvinylcaprolactam und Copolymeren von Vinylcaprolactam und einem Vinylmonomer der Formel (I), die in Anspruch 9 angegeben ist, oder einem Monomer, das unter Maleinsäureanhydrid, Itaconsäure, Vinylpyrrolidon, Styrol und seinen Derivaten, Dimethyldiallylammoniumchlorid, Vinylacetamid, Vinylethern und Derivaten von Vinylacetat ausgewählt ist.

30. Emulsion nach einem der Ansprüche 23 bis 28, wobei die LCST-Einheiten aus Polypropylenoxid der Formel (PPO)ₙ bestehen, wobei n eine ganze Zahl von 10 bis 50 bedeutet, oder statistischen Copolymeren von Ethylenoxid (EO) und Propylenoxid (PO), die durch die folgende Formel dargestellt werden:
(EO)ₘ (PO)ₙ,
wobei m eine ganze Zahl von 1 bis 40 und vorzugsweise 2 bis 20 bedeutet und n eine ganze Zahl von 10 bis 60 und vorzugsweise 20 bis 50 ist.

31. Emulsion nach Anspruch 30, wobei die Molmasse der LCST-Einheiten 500 bis 5 300 g/mol und vorzugsweise 1 500 bis 4 000 g/mol beträgt.

32. Schaumbildende Zusammensetzung nach Anspruch 29, wobei die LCST-Einheiten gebildet werden von Poly-N-Isopropylacrylamid oder Poly-N-Ethylacrylamid und den Copolymeren von N-Isopropylacrylamid oder N-Ethylacrylamid und einem Vinylmonomer, das der in Anspruch 9 angegebenen Formel (I) entspricht, oder einem Monomer, das unter Maleinsäureanhydrid, Itaconsäure, Vinylpyrrolidon, Styrol und seinen Derivaten, Dimethyldiallylammoniumchlorid, Vinylacetamid, Vinylalkohol, Vinylacetat, Vinylethern und Vinylacetatderivaten ausgewählt ist.

33. Emulsion nach Anspruch 32, wobei die Molmasse der LCST-Einheiten 1 000 bis 500 000 g/mol und vorzugsweise 2 000 bis 50 000 g/mol beträgt.

34. Emulsion nach einem der Ansprüche 23 bis 28, wobei die LCST-Einheiten aus einem Polyvinylcaprolactam oder einem Copolymer von Vinylcaprolactam und einem Vinylmonomer der in Anspruch 9 angegebenen Formel (I) oder einem Monomer gebildet sind, das unter Maleinsäureanhydrid, Itaconsäure, Vinylpyrrolidon, Styrol und seinen Derivaten, Dimethyldiallylammoniumchlorid, Vinylacetamid, Vinylalkohol, Vinylacetat, Vinylethern und Derivaten von Vinylacetat ausgewählt ist.

35. Emulsion nach Anspruch 34, wobei die Molmasse der LCST-Einheiten 1 000 bis 500 000 g/mol und vorzugsweise 2 000 bis 50 000 g/mol beträgt.

36. Emulsion nach einem der Ansprüche 23 bis 35, wobei der Masseanteil der LCST-Einheiten des Polymers 5 bis 70 %, vorzugsweise 20 bis 65 % und besser 30 bis 60 %, bezogen auf das Polymer, beträgt.

37. Emulsion nach einem der Ansprüche 23 bis 36, wobei die Entmischungstemperatur der LCST-Einheiten für eine Massekonzentration der LCST-Einheiten in Wasser von 1 % im Bereich von 5 bis 40°C liegt.

38. Emulsion nach einem der Ansprüche 23 bis 37, wobei die Massekonzentration des Polymers der wässrigen Phase 5 % oder darunter und vorzugsweise 0,01 bis 5 % beträgt.

39. Emulsion nach einem der Ansprüche 23 bis 38, wobei die wässrige Phase ferner einen emulgierenden grenzflächenaktiven Stoff in einer Massekonzentration enthält, die 1 % nicht übersteigt.

40. Emulsion nach einem der Ansprüche 23 bis 39, die ferner einen Gelbildner enthält.

41. Kosmetische Verwendung der schaumbildenden Zusammensetzung nach einem der Ansprüche 7 bis 22 für die Reinigung und/oder zum Abschminken der Haut einschließlich der Kopfhaut, der Nägel, der Haare, der Wimpern, der Augenbrauen, der Augen, der Schleimhäute und der Semischleimhäute und aller anderen Hautbereiche des Körpers und des Gesichts.

42. Kosmetische Verwendung einer kosmetischen Emulsion nach einem der Ansprüche 23 bis 40 für die Behandlung, für die Pflege, zum Schutz und/oder zum Schminken der Haut des Gesichts und/oder des Körpers, der Schleimhäute (Lippen), der Kopfhaut und/oder der Kerstinfasern.

43. Kosmetisches Verfahren für die Reinigung und/oder zum Abschminken der Haut, der Kopfhaut und/oder der Haare, **dadurch gekennzeichnet, dass** auf die Haut, die Kopfhaut und/oder die Haare die erfindungsgemäße Zusammensetzung in Gegenwart von Wasser aufgetragen wird und der gebildete Schaum und die Schmutzrückstände durch Spülen mit Wasser entfernt werden.
